# EUROPEAN PATENT APPLICATION

(11) **EP 3 903 790 A1**
(43) Date of publication of application: **03.11.2021**
(21) Application number: 19902517.2
(22) Date of filing: 27.12.2019
(51) Int. Cl.: A61K 31/695, A61P 35/00, C07K 16/30, C07K 19/00, A61K 47/42, A61K 47/54, C12N 15/13, C12N 15/31, C12N 15/62, G01N 33/531, G01N 33/574

(54) **FUSION PROTEIN OF STREPTAVIDIN VARIANT AND ANTIBODY RECOGNIZING CANCER CELLS**

(30) Priority: 28.12.2018 JP 2018247363
(71) Applicant: The University of Tokyo, Bunkyo-ku, Tokyo 113-8654 (JP); Savid Therapeutics Inc., Tokyo 168-0064 (JP)
(72) Inventor: TANAKA Toshiya, Tokyo 113-8654 (JP); KODAMA Tatsuhiko, Tokyo 113-8654 (JP); YAMASHITA Takefumi, Tokyo 113-8654 (JP); KANAI Motomu, Tokyo 113-8654 (JP); YAMATSUGU Kenzo, Tokyo 113-8654 (JP); TATSUMI Toshifumi, Tokyo 113-8654 (JP); TAKAHASHI Kazuki, Tokyo 113-8654 (JP); ISHIKAWA Shumpei, Tokyo 113-8654 (JP); SUGIYAMA Akira, Tokyo 113-8654 (JP); TSUKAGOSHI Masanobu, Tokyo 168-0064 (JP)
(74) Representative: Godemeyer Blum Lenze Patentanwälte Partnerschaft mbB - werkpatent
(86) International application number: PCT/JP2019/051449
(87) International publication number: WO 2020/138427

(57) **Abstract**

It is an object of the present invention to provide a fusion protein of an antibody that recognizes cancer cells and a mutant streptavidin, which is for use in the treatment or diagnosis of cancer. According to the present invention, provided is a fusion protein having the amino acid sequence as set forth in SEQ ID NO: 2 or SEQ ID NO: 7, a linker sequence, and the amino acid sequence as set forth in SEQ ID NO: 1 (provided that the amino acid sequence portion consisting of 6 histidine residues at the C-terminus thereof may be partially or entirely deleted), from the N-terminal side to the C-terminal side, in this order.

SEQUENCE LISTING
<110>  Tokyo University
<110>  Savid Therapeutics Inc
<120>  Fused protein of an antibody which recognizes cancer-cells and a
streptavidin mutant
<130>  191598A
<160>  18
<170>  PatentIn version 3.5
<210>  1
<211>  133
<212>  PRT
<213> Artificial Sequence
<220>
<223> Description of Artificial Sequence: Recombinant protein
<400>  1
Ala Glu Ala Gly Ile Thr Gly Thr Trp Ser Asp Gln Leu Gly Asp Thr
1               5                   10                  15
Phe Ile Val Thr Ala Gly Ala Asp Gly Ala Leu Thr Gly Thr Tyr Glu
            20                  25                  30
Asn Ala Val Gly Gly Ala Glu Ser Arg Tyr Val Leu Thr Gly Arg Tyr
        35                  40                  45
Asp Ser Ala Pro Ala Thr Asp Gly Ser Gly Thr Ala Leu Gly Trp Thr
    50                  55                  60
Val Ala Trp Lys Asn Asn Ser Lys Asn Ala His Ser Ala Thr Thr Trp
65                  70                  75                  80
Ser Gly Gln Tyr Val Gly Gly Ala Asp Ala Lys Ile Asn Thr Gln Trp
                85                  90                  95
Leu Leu Thr Ser Gly Thr Thr Asn Ala Asn Ala Trp Lys Ser Thr Leu
            100                 105                 110
Val Gly His Asp Thr Phe Thr Lys Val Lys Pro Ser Ala Ala Ser His
        115                 120                 125
His His His His His
    130
<210>  2
<211>  241
<212>  PRT
<213> Artificial Sequence
<220>
<223> Description of Artificial Sequence: Recombinant protein
<400>  2
Gln Val Lys Leu Glu Gln Ser Gly Ala Glu Val Val Lys Pro Gly Ala
1               5                   10                  15
Ser Val Lys Leu Ser Cys Lys Ala Ser Gly Phe Asn Ile Lys Asp Ser
            20                  25                  30
Tyr Met His Trp Leu Arg Gln Gly Pro Gly Gln Arg Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ala Asn Thr Ala Tyr
65                  70                  75                  80
Leu Gly Leu Ser Ser Leu Arg Pro Glu Asp Thr Ala Val Tyr Tyr Cys
                85                  90                  95
Asn Glu Gly Thr Pro Thr Gly Pro Tyr Tyr Phe Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Glu Asn Val Leu Thr Gln Ser Pro Ser
    130                 135                 140
Ser Met Ser Val Ser Val Gly Asp Arg Val Asn Ile Ala Cys Ser Ala
145                 150                 155                 160
Ser Ser Ser Val Pro Tyr Met His Trp Leu Gln Gln Lys Pro Gly Lys
                165                 170                 175
Ser Pro Lys Leu Leu Ile Tyr Leu Thr Ser Asn Leu Ala Ser Gly Val

                180                 185                 190
Pro Ser Arg Phe Ser Gly Ser Gly Ser Gly Thr Asp Tyr Ser Leu Thr
        195                 200                 205
Ile Ser Ser Val Gln Pro Glu Asp Ala Ala Thr Tyr Tyr Cys Gln Gln
    210                 215                 220
Arg Ser Ser Tyr Pro Leu Thr Phe Gly Gly Gly Thr Lys Leu Glu Ile
225                 230                 235                 240
Lys

<210>  3
<211>  383
<212>  PRT
<213> Artificial Sequence
<220>
<223> Description of Artificial Sequence: Recombinant protein
<400>  3
Gln Val Lys Leu Glu Gln Ser Gly Ala Glu Val Val Lys Pro Gly Ala
1               5                   10                  15
Ser Val Lys Leu Ser Cys Lys Ala Ser Gly Phe Asn Ile Lys Asp Ser
            20                  25                  30
Tyr Met His Trp Leu Arg Gln Gly Pro Gly Gln Arg Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ala Asn Thr Ala Tyr
65                  70                  75                  80
Leu Gly Leu Ser Ser Leu Arg Pro Glu Asp Thr Ala Val Tyr Tyr Cys
                85                  90                  95
Asn Glu Gly Thr Pro Thr Gly Pro Tyr Tyr Phe Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Glu Asn Val Leu Thr Gln Ser Pro Ser
    130                 135                 140
Ser Met Ser Val Ser Val Gly Asp Arg Val Asn Ile Ala Cys Ser Ala
145                 150                 155                 160
Ser Ser Ser Val Pro Tyr Met His Trp Leu Gln Gln Lys Pro Gly Lys
                165                 170                 175
Ser Pro Lys Leu Leu Ile Tyr Leu Thr Ser Asn Leu Ala Ser Gly Val
            180                 185                 190
Pro Ser Arg Phe Ser Gly Ser Gly Ser Gly Thr Asp Tyr Ser Leu Thr
        195                 200                 205
Ile Ser Ser Val Gln Pro Glu Asp Ala Ala Thr Tyr Tyr Cys Gln Gln
    210                 215                 220
Arg Ser Ser Tyr Pro Leu Thr Phe Gly Gly Gly Thr Lys Leu Glu Ile
225                 230                 235                 240
Lys Gly Gly Gly Gly Ser Gly Gly Gly Gly Ala Glu Ala Gly Ile Thr
                245                 250                 255
Gly Thr Trp Ser Asp Gln Leu Gly Asp Thr Phe Ile Val Thr Ala Gly
            260                 265                 270
Ala Asp Gly Ala Leu Thr Gly Thr Tyr Glu Asn Ala Val Gly Gly Ala
        275                 280                 285
Glu Ser Arg Tyr Val Leu Thr Gly Arg Tyr Asp Ser Ala Pro Ala Thr
    290                 295                 300
Asp Gly Ser Gly Thr Ala Leu Gly Trp Thr Val Ala Trp Lys Asn Asn
305                 310                 315                 320
Ser Lys Asn Ala His Ser Ala Thr Thr Trp Ser Gly Gln Tyr Val Gly
                325                 330                 335
Gly Ala Asp Ala Lys Ile Asn Thr Gln Trp Leu Leu Thr Ser Gly Thr
            340                 345                 350
Thr Asn Ala Asn Ala Trp Lys Ser Thr Leu Val Gly His Asp Thr Phe
        355                 360                 365
Thr Lys Val Lys Pro Ser Ala Ala Ser His His His His His His

370                 375                 380
<210>  4
<211>  405
<212>  PRT
<213> Artificial Sequence
<220>
<223> Description of Artificial Sequence: Recombinant protein
<400>  4
Met Lys Tyr Leu Leu Pro Thr Ala Ala Ala Gly Leu Leu Leu Leu Ala
1               5                   10                  15
Ala Gln Pro Ala Met Ala Gln Val Lys Leu Glu Gln Ser Gly Ala Glu
            20                  25                  30
Val Val Lys Pro Gly Ala Ser Val Lys Leu Ser Cys Lys Ala Ser Gly
        35                  40                  45
Phe Asn Ile Lys Asp Ser Tyr Met His Trp Leu Arg Gln Gly Pro Gly
    50                  55                  60
Gln Arg Leu Glu Trp Ile Gly Trp Ile Asp Pro Glu Asn Gly Asp Thr
65                  70                  75                  80
Glu Tyr Ala Pro Lys Phe Gln Gly Lys Ala Thr Phe Thr Thr Asp Thr
                85                  90                  95
Ser Ala Asn Thr Ala Tyr Leu Gly Leu Ser Ser Leu Arg Pro Glu Asp
            100                 105                 110
Thr Ala Val Tyr Tyr Cys Asn Glu Gly Thr Pro Thr Gly Pro Tyr Tyr
        115                 120                 125
Phe Asp Tyr Trp Gly Gln Gly Thr Leu Val Thr Val Ser Ser Gly Gly
    130                 135                 140
Gly Gly Ser Gly Gly Gly Gly Ser Gly Gly Gly Gly Ser Glu Asn Val
145                 150                 155                 160
Leu Thr Gln Ser Pro Ser Ser Met Ser Val Ser Val Gly Asp Arg Val
                165                 170                 175
Asn Ile Ala Cys Ser Ala Ser Ser Ser Val Pro Tyr Met His Trp Leu
            180                 185                 190
Gln Gln Lys Pro Gly Lys Ser Pro Lys Leu Leu Ile Tyr Leu Thr Ser
        195                 200                 205
Asn Leu Ala Ser Gly Val Pro Ser Arg Phe Ser Gly Ser Gly Ser Gly
    210                 215                 220
Thr Asp Tyr Ser Leu Thr Ile Ser Ser Val Gln Pro Glu Asp Ala Ala
225                 230                 235                 240
Thr Tyr Tyr Cys Gln Gln Arg Ser Ser Tyr Pro Leu Thr Phe Gly Gly
                245                 250                 255
Gly Thr Lys Leu Glu Ile Lys Gly Gly Gly Gly Ser Gly Gly Gly Gly
            260                 265                 270
Ala Glu Ala Gly Ile Thr Gly Thr Trp Ser Asp Gln Leu Gly Asp Thr
        275                 280                 285
Phe Ile Val Thr Ala Gly Ala Asp Gly Ala Leu Thr Gly Thr Tyr Glu
    290                 295                 300
Asn Ala Val Gly Gly Ala Glu Ser Arg Tyr Val Leu Thr Gly Arg Tyr
305                 310                 315                 320
Asp Ser Ala Pro Ala Thr Asp Gly Ser Gly Thr Ala Leu Gly Trp Thr
                325                 330                 335
Val Ala Trp Lys Asn Asn Ser Lys Asn Ala His Ser Ala Thr Thr Trp
            340                 345                 350
Ser Gly Gln Tyr Val Gly Gly Ala Asp Ala Lys Ile Asn Thr Gln Trp
        355                 360                 365
Leu Leu Thr Ser Gly Thr Thr Asn Ala Asn Ala Trp Lys Ser Thr Leu
    370                 375                 380
Val Gly His Asp Thr Phe Thr Lys Val Lys Pro Ser Ala Ala Ser His
385                 390                 395                 400
His His His His His
                405
<210>  5
<211>  1218
<212>  DNA

<213> Artificial Sequence
<220>
<223> Description of Artificial Sequence: Recombinant DNA
<400> 5
atgaaatatc tgctgccgac cgcagcagcg ggtctgctgc tgctggcagc acagcctgca 60
atggcacagg ttaaactgga acagagcggt gccgaagttg ttaaaccggg tgcaagcgtt 120
aaactgagct gtaaagcaag cggctttaac atcaaagata gctatatgca ttggctgcgt 180
cagggtccgg gtcagcgtct ggaatggatt ggttggattg atccggaaaa tggtgatacc 240
gaatatgcac cgaaatttca gggtaaagca acctttacca ccgataccag cgcaaatacc 300
gcatatctgg gtctgagcag cctgcgtccg gaagataccg cagtgtatta ttgtaatgaa 360
ggcaccccga ccggtccgta ttatttcgat tattggggtc agggcaccct ggttaccgtt 420
agcagcggtg gtggtggtag tggtggcggt ggttcaggcg gtggcggtag cgaaaatgtt 480
ctgacccaga gcccgagcag catgagcgtt agcgttggtg atcgtgttaa tattgcatgt 540
agcgcaagca gcagcgttcc gtacatgcac tggctgcagc agaaaccggg taaaagcccg 600
aaactgctga tttatctgac cagcaatctg gcaagcggtg ttccgagccg ttttagcggt 660
agcggtagtg gcaccgatta tagcctgacc attagcagcg tgcagcctga agatgcagca 720
acctattatt gtcagcagcg tagcagttat ccgctgacct ttggtggtgg caccaaactg 780
gaaattaaag ggggtggtgg ctcaggtggc ggaggtgcag aagcaggtat taccggtaca 840
tggtcagatc agctgggtga tacctttatt gttaccgcag gcgcagatgg tgcactgacc 900
ggcacctatg aaaatgcagt tggtggtgca gaaagccgtt atgtgctgac cggtcgttat 960
gatagcgcac cggcaaccga tggtagcggc accgcactgg gttggaccgt tgcatggaaa 1020
aataacagca aaaatgcaca tagcgcaacc acctggtcag gtcagtatgt gggtggtgcc 1080
gatgccaaaa ttaacaccca gtggctgctg accagcggta caaccaatgc aaatgcctgg 1140
aaaagtaccc tggttggtca tgatacattc accaaagtta aaccgagcgc agcaagccat 1200
catcatcacc atcattaa 1218
<210> 6
<211> 141
<212> PRT
<213> Artificial Sequence
<220>
<223> Description of Artificial Sequence: Recombinant protein
<400> 6
Met Asp Lys Ile Ala Ile Val Asn Met Gly Ser Leu Phe Gln Gln Val
1               5                   10                  15
Ala Gln Lys Thr Gly Val Ser Asn Thr Leu Glu Asn Glu Phe Lys Gly
            20                  25                  30
Arg Ala Ser Glu Leu Gln Arg Met Glu Thr Asp Leu Gln Ala Lys Met
        35                  40                  45
Lys Lys Leu Gln Ser Met Lys Ala Gly Ser Asp Arg Thr Lys Leu Glu
    50                  55                  60
Lys Asp Val Met Ala Gln Arg Gln Thr Phe Ala Gln Lys Ala Gln Ala
65                  70                  75                  80
Phe Glu Gln Asp Arg Ala Arg Arg Ser Asn Glu Glu Arg Gly Lys Leu
                85                  90                  95
Val Thr Arg Ile Gln Thr Ala Val Lys Ser Val Ala Asn Ser Gln Asp
            100                 105                 110
Ile Asp Leu Val Val Asp Ala Asn Ala Val Ala Tyr Asn Ser Ser Asp
        115                 120                 125
Val Lys Asp Ile Thr Ala Asp Val Leu Lys Gln Val Lys
    130                 135                 140
<210> 7
<211> 247
<212> PRT
<213> Artificial Sequence
<220>
<223> Description of Artificial Sequence: Recombinant protein
<400> 7
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Asn Ile Lys Asp Thr
            20                  25                  30
Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ser Arg Trp Gly Gly Asp Gly Phe Tyr Ala Met Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Gly Gly Gly Gly Ser Asp Ile Gln Met
    130                 135                 140
Thr Gln Ser Pro Ser Ser Leu Ser Ala Ser Val Gly Asp Arg Val Thr
145                 150                 155                 160
Ile Thr Cys Arg Ala Ser Gln Asp Val Asn Thr Ala Val Ala Trp Tyr
                165                 170                 175
Gln Gln Lys Pro Gly Lys Ala Pro Lys Leu Leu Ile Tyr Ser Ala Ser
            180                 185                 190
Phe Leu Tyr Ser Gly Val Pro Ser Arg Phe Ser Gly Ser Arg Ser Gly
        195                 200                 205
Thr Asp Phe Thr Leu Thr Ile Ser Ser Leu Gln Pro Glu Asp Phe Ala
    210                 215                 220
Thr Tyr Tyr Cys Gln Gln His Tyr Thr Thr Pro Pro Thr Phe Gly Gln
225                 230                 235                 240
Gly Thr Lys Val Glu Ile Lys
                245
<210>  8
<211>  391
<212>  PRT
<213> Artificial Sequence
<220>
<223> Description of Artificial Sequence: Recombinant protein
<400>  8
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Asn Ile Lys Asp Thr
               20                  25                  30
   Tyr Ile His Trp Val Arg Gln Ala Pro Gly Lys Gly Leu Glu Trp Val
           35                  40                  45
   Ala Arg Ile Tyr Pro Thr Asn Gly Tyr Thr Arg Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ser Arg Trp Gly Gly Asp Gly Phe Tyr Ala Met Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gly Gly Gly Gly Ser Asp Ile Gln Met
       130                 135                 140
   Thr Gln Ser Pro Ser Ser Leu Ser Ala Ser Val Gly Asp Arg Val Thr
   145                 150                 155                 160
   Ile Thr Cys Arg Ala Ser Gln Asp Val Asn Thr Ala Val Ala Trp Tyr
                   165                 170                 175
   Gln Gln Lys Pro Gly Lys Ala Pro Lys Leu Leu Ile Tyr Ser Ala Ser
               180                 185                 190
   Phe Leu Tyr Ser Gly Val Pro Ser Arg Phe Ser Gly Ser Arg Ser Gly
           195                 200                 205
   Thr Asp Phe Thr Leu Thr Ile Ser Ser Leu Gln Pro Glu Asp Phe Ala
       210                 215                 220
   Thr Tyr Tyr Cys Gln Gln His Tyr Thr Thr Pro Pro Thr Phe Gly Gln
   225                 230                 235                 240
   Gly Thr Lys Val Glu Ile Lys Gly Gly Gly Gly Gly Ser Gly Gly Gly
                   245                 250                 255
   Gly Gly Ala Glu Ala Gly Ile Thr Gly Thr Trp Ser Asp Gln Leu Gly
               260                 265                 270
   Asp Thr Phe Ile Val Thr Ala Gly Ala Asp Gly Ala Leu Thr Gly Thr
           275                 280                 285
   Tyr Glu Asn Ala Val Gly Gly Ala Glu Ser Arg Tyr Val Leu Thr Gly
       290                 295                 300
   Arg Tyr Asp Ser Ala Pro Ala Thr Asp Gly Ser Gly Thr Ala Leu Gly
   305                 310                 315                 320
   Trp Thr Val Ala Trp Lys Asn Asn Ser Lys Asn Ala His Ser Ala Thr
                   325                 330                 335
   Thr Trp Ser Gly Gln Tyr Val Gly Gly Ala Asp Ala Lys Ile Asn Thr
               340                 345                 350
   Gln Trp Leu Leu Thr Ser Gly Thr Thr Asn Ala Asn Ala Trp Lys Ser
           355                 360                 365
   Thr Leu Val Gly His Asp Thr Phe Thr Lys Val Lys Pro Ser Ala Ala
       370                 375                 380
   Ser His His His His His His
   385                 390
<210>  9
<211>  413
<212>  PRT
<213> Artificial Sequence
<220>
<223> Description of Artificial Sequence: Recombinant protein
<400>  9
   Met Lys Tyr Leu Leu Pro Thr Ala Ala Ala Gly Leu Leu Leu Leu Ala
   1               5                   10                  15
   Ala Gln Pro Ala Met Ala Glu Val Gln Leu Val Glu Ser Gly Gly Gly
               20                  25                  30
   Leu Val Gln Pro Gly Gly Ser Leu Arg Leu Ser Cys Ala Ala Ser Gly
           35                  40                  45
   Phe Asn Ile Lys Asp Thr Tyr Ile His Trp Val Arg Gln Ala Pro Gly
       50                  55                  60
   Lys Gly Leu Glu Trp Val Ala Arg Ile Tyr Pro Thr Asn Gly Tyr Thr
   65                  70                  75                  80
   Arg Tyr Ala Asp Ser Val Lys Gly Arg Phe Thr Ile Ser Ala Asp Thr
                   85                  90                  95
   Ser Lys Asn Thr Ala Tyr Leu Gln Met Asn Ser Leu Arg Ala Glu Asp
               100                 105                 110
   Thr Ala Val Tyr Tyr Cys Ser Arg Trp Gly Gly Asp Gly Phe Tyr Ala
           115                 120                 125
   Met Asp Tyr Trp Gly Gln Gly Thr Leu Val Thr Val Ser Ser Gly Gly
       130                 135                 140
   Gly Gly Ser Gly Gly Gly Gly Ser Gly Gly Gly Gly Ser Gly Gly Gly
   145                 150                 155                 160
   Gly Ser Asp Ile Gln Met Thr Gln Ser Pro Ser Ser Leu Ser Ala Ser
                   165                 170                 175
   Val Gly Asp Arg Val Thr Ile Thr Cys Arg Ala Ser Gln Asp Val Asn
               180                 185                 190
   Thr Ala Val Ala Trp Tyr Gln Gln Lys Pro Gly Lys Ala Pro Lys Leu
           195                 200                 205
   Leu Ile Tyr Ser Ala Ser Phe Leu Tyr Ser Gly Val Pro Ser Arg Phe
       210                 215                 220
   Ser Gly Ser Arg Ser Gly Thr Asp Phe Thr Leu Thr Ile Ser Ser Leu
   225                 230                 235                 240
   Gln Pro Glu Asp Phe Ala Thr Tyr Tyr Cys Gln Gln His Tyr Thr Thr
                   245                 250                 255
   Pro Pro Thr Phe Gly Gln Gly Thr Lys Val Glu Ile Lys Gly Gly Gly
               260                 265                 270
   Gly Gly Ser Gly Gly Gly Gly Gly Ala Glu Ala Gly Ile Thr Gly Thr
           275                 280                 285
   Trp Ser Asp Gln Leu Gly Asp Thr Phe Ile Val Thr Ala Gly Ala Asp
   290 295 300
   Gly Ala Leu Thr Gly Thr Tyr Glu Asn Ala Val Gly Gly Ala Glu Ser
   305 310 315 320
   Arg Tyr Val Leu Thr Gly Arg Tyr Asp Ser Ala Pro Ala Thr Asp Gly
   325 330 335
   Ser Gly Thr Ala Leu Gly Trp Thr Val Ala Trp Lys Asn Asn Ser Lys
   340 345 350
   Asn Ala His Ser Ala Thr Thr Trp Ser Gly Gln Tyr Val Gly Gly Ala
   355 360 365
   Asp Ala Lys Ile Asn Thr Gln Trp Leu Leu Thr Ser Gly Thr Thr Asn
   370 375 380
   Ala Asn Ala Trp Lys Ser Thr Leu Val Gly His Asp Thr Phe Thr Lys
   385 390 395 400
   Val Lys Pro Ser Ala Ala Ser His His His His His His
   405 410
<210>  10
<211>  1242
<212>  DNA
<213>  Artificial Sequence
<220>
<223>  Description of Artificial Sequence: Recombinant DNA
<400>  10
atgaaatatc tgctgccgac cgcagcagcg ggtctgctgc tgctggcagc acagcctgca      60
atggcagaag ttcagctggt tgaaagcggt ggtggtctgg ttcagcctgg tggtagcctg     120
cgtctgagct gtgcagcaag cggttttaac attaaagata cctatattca ttgggtgcgt     180
caggcacctg gtaaaggtct ggaatgggtt gcacgtattt atccgaccaa tggttatacc     240
cgttatgccg atagcgttaa aggtcgtttt accattagcg cagataccag caaaaatacc     300
gcatacctgc agatgaatag tctgcgtgca gaggataccg cagtgtatta ttgtagccgt     360
tggggtggtg atggttttta tgcaatggat tattggggtc agggcaccct ggttaccgtt     420
agctcaggtg gaggcggttc cggtggcgga ggttccggtg gaggtggctc cggtggcgga     480
ggttccgata ttcagatgac ccagagtccg agcagcctga gcgcaagcgt tggtgatcgt     540
gtgaccatta cctgtcgtgc aagccaggat gttaatacag cagttgcatg gtatcagcag     600
aaaccgggta aagcaccgaa actgctgatt tatagcgcaa gctttctgta tagcggtgtt     660
ccgagccgtt ttagcggtag ccgtagcggc accgatttta ccctgaccat tagcagcctg     720
cagccggaag attttgcaac ctattattgt cagcagcatt acaccacacc gcctaccttt     780
ggccagggca ccaaagttga aattaaagga ggtggcggtg gatccggcgg aggtggcgga     840
gcagaagcag gtattaccgg tacatggtca gatcagctgg gtgatacctt tattgttacc     900
gcaggcgcag atggtgcact gaccggcacc tatgaaaatg cagttggtgg tgcagaaagc     960
cgttatgtgc tgaccggtcg ttatgatagc gcaccggcaa ccgatggtag cggcaccgca    1020
ctgggttgga ccgttgcatg gaaaaataac agcaaaaatg cacatagcgc aaccacctgg    1080
tcaggtcagt atgtgggtgg tgccgatgcc aaaattaaca cccagtggct gctgaccagc    1140
ggtacaacca atgcaaatgc ctggaaaagt accctggttg gtcatgatac attcaccaaa    1200
gttaaaccga gcgcagcaag ccatcatcat caccatcatt aa                       1242
<210>  11
<211>  9
<212>  PRT
<213>  Artificial Sequence
<220>
<223>  Description of Artificial Sequence: Recombinant linker
<400>  11
Gly Gly Gly Gly Ser Gly Gly Gly Gly
1               5
<210>  12
<211>  40
<212>  DNA
<213>  Artificial Sequence
<220>
<223>  Description of Artificial Sequence: Synthesized DNA
<400>  12
aaggagatat acatatggat aaaattgcca ttgttaatat                            40
<210>  13
<211>  39

<212>  DNA
<213> Artificial Sequence
<220>
<223> Description of Artificial Sequence: Synthesized DNA
<400>  13
ttgagatctg ccatatgtta tttcacttgt ttcagaacg                              39
<210>  14
<211>  35
<212>  DNA
<213> Artificial Sequence
<220>
<223> Description of Artificial Sequence: Synthesized DNA
<400>  14
agaaggagat ataccatgaa atatctgctg ccgac                                  35
<210>  15
<211>  36
<212>  DNA
<213> Artificial Sequence
<220>
<223> Description of Artificial Sequence: Synthesized DNA
<400>  15
cgccgagctc gaattttaat gatggtgatg atgatg                                 36
<210>  16
<211>  26
<212>  DNA
<213> Artificial Sequence
<220>
<223> Description of Artificial Sequence: Synthesized DNA
<400>  16
ggtatatctc cttcttaaag ttaaac                                            26
<210>  17
<211>  24
<212>  DNA
<213> Artificial Sequence
<220>
<223> Description of Artificial Sequence: Synthesized DNA
<400>  17
aattcgagct cggcgcgcct gcag                                              24
<210>  18
<211>  11
<212>  PRT
<213> Artificial Sequence
<220>
<223> Description of Artificial Sequence: Recombinant linker
<400>  18
Gly Gly Gly Gly Gly Ser Gly Gly Gly Gly Gly
1               5                   10

## Description

### Technical Field

The present invention relates to a fusion protein of an antibody that recognizes cancer cells and a mutant streptavidin, and the use thereof.

### Background Art

Avidin and biotin, or streptavidin and biotin have an extremely high affinity between them (Kd = 10⁻¹⁵ to 10⁻¹⁴ M). This is one of extremely strong interactions between two biomolecules. At present, the interaction between avidin/streptavidin and biotin has been widely applied in the field of biochemistry, molecular biology, or medicine. A drug delivery method and a pretargeting method, in which high binding ability between avidin/streptavidin and biotin is combined with an antibody molecule, have been devised. In connection with these studies, a mutant streptavidin with a reduced affinity for natural biotin and a biotin-modified dimer having a high affinity for the mutant streptavidin with a low affinity for natural biotin are reported in Patent Document 1.

### Prior Art Documents

### Patent Documents

Patent Document 1: International Publication WO2015/125820

### Summary of Invention

### Object to be Solved by the Invention

It is an object of the present invention to provide a fusion protein of an antibody that recognizes cancer cells and a mutant streptavidin, which is for use in the treatment or diagnosis of cancer. It is another object of the present invention to provide a means for treating cancer or a means for diagnosing cancer, in which the above-described fusion protein is used.

### Means for Solving the Object

As a result of intensive studies directed towards achieving the above-described objects, the present inventor has selected an anti-CEA antibody and an anti-HER2 antibody as antibodies that recognize cancer cells, and then, have prepared a fusion protein of the above-described antibody and a mutant streptavidin. Thereafter, the present inventor has found that the proliferation of cancer cells can be suppressed by photoimmunotherapy using the above-described fusion protein and a conjugate of a biotin-modified dimer and a phthalocyanine dye, thereby completing the present invention.

Specifically, according to the present invention, the following inventions are provided.
<1> A fusion protein having the amino acid sequence as set forth in SEQ ID NO: 2 or SEQ ID NO: 7, a linker sequence, and the amino acid sequence as set forth in SEQ ID NO: 1 (provided that the amino acid sequence portion consisting of 6 histidine residues at the C-terminus thereof may be partially or entirely deleted), from the N-terminal side to the C-terminal side, in this order.
<2> The fusion protein according to <1>, wherein the number of amino acids of the linker sequence is 5 to 15.
<3> The fusion protein according to <1> or <2>, wherein the linker sequence consists of 4 to 14 glycine residues and 1 cysteine residue.
<4> The fusion protein according to any one of <1> to <3>, wherein the linker sequence is Gly-Gly-Gly-Gly-Ser-Gly-Gly-Gly-Gly or Gly-Gly-Gly-Gly-Gly-Ser-Gly-Gly-Gly-Gly-Gly.
<5> A fusion protein having the amino acid sequence as set forth in SEQ ID NO: 3 or SEQ ID NO: 8 (provided that the amino acid sequence portion consisting of 6 histidine residues at the C-terminus thereof may be partially or entirely deleted).
<6> The fusion protein according to any one of <1> to <5>, further having a secretory signal sequence.
<7> A fusion protein having the amino acid sequence as set forth in SEQ ID NO: 4 or SEQ ID NO: 9 (provided that the amino acid sequence portion consisting of 6 histidine residues at the C-terminus thereof may be partially or entirely deleted).
<8> A nucleic acid encoding a fusion protein having the amino acid sequence as set forth in SEQ ID NO: 3 or SEQ ID NO: 8 (provided that the amino acid sequence portion consisting of 6 histidine residues at the C-terminus thereof may be partially or entirely deleted).
<9> A nucleic acid encoding a fusion protein having the amino acid sequence as set forth in SEQ ID NO: 4 or SEQ ID NO: 9 (provided that the amino acid sequence portion consisting of 6 histidine residues at the C-terminus thereof may be partially or entirely deleted).
<10> A cancer therapeutic agent or a cancer diagnostic agent, comprising the fusion protein according to any one of <1> to <7>.
<11> A kit for treating or diagnosing cancer, comprising (1) the fusion protein according to any one of <1> to <7>, and (2) a conjugate of a compound represented by the following formula (1) or a salt thereof and a diagnostic substance or a therapeutic substance: 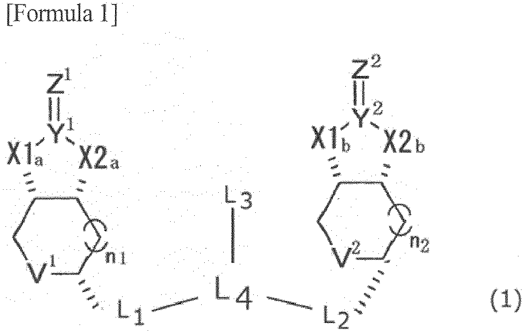 wherein
   X1a, X1b, X2a and X2b each independently represent O or NH,
   Y¹ and Y² each independently represent C or S,
   Z¹ and Z² each independently represent O, S or NH,
   V¹ and V² each independently represent S or S⁺-O⁻,
   n1 and n2 each independently represent an integer of 0 or 1,
   L₁ and L₂ each independently represent a divalent linking group,
   L₃ represents a group comprising a functional group capable of binding to the diagnostic substance or the therapeutic substance at the terminus, and
   L₄ represents a trivalent linking group.
<12> The kit according to <11>, wherein the diagnostic substance or the therapeutic substance is a phthalocyanine dye.

### Advantageous Effects of Invention

The proliferation of cancer cells can be suppressed by using the fusion protein of the present invention consisting of an antibody that recognizes cancer cells and a mutant streptavidin.

### Brief Description of Drawings

[Fig. 1] Fig. 1 shows an outline of a domain structure.
[Fig. 2] Fig. 2 shows a CBB-stained SDS-PAGE electrophoretic pattern of CEA-V2122.
[Fig. 3] Fig. 3 shows evaluation of the binding performance of CEA-Cupid with an antigen (CEACAM5).
[Fig. 4] Fig. 4 shows evaluation of the binding performance of CEA-Cupid with a modified biotin.
[Fig. 5] Fig. 5 shows stained cell images obtained using FITC-labeled CEA-V2122.
[Fig. 6] Fig. 6 shows time-series data of stained MKN45 cell images obtained using FITC-labeled CEA-V2122.
[Fig. 7] Fig. 7 shows photoactivatable compound-binding modified biotins.
[Fig. 8] Fig. 8 shows *in vitro* cytotoxicity obtained by a combination of CEA-Cupid and a photoactivatable compound-binding modified biotin.
[Fig. 9] Fig. 9 shows a change in the size of a tumor mass.
[Fig. 10] Fig. 10 shows *in vivo* cytotoxicity obtained by a combination of CEA-Cupid and a photoactivatable compound-binding modified biotin.
[Fig. 11] Fig. 11 shows histopathological images of *in vivo* cytotoxicity obtained by a combination of CEA-Cupid and a photoactivatable compound-binding modified biotin.
[Fig. 12] Fig. 12 shows the analysis of pathological sections using an anti-CEACAM5 antibody.
[Fig. 13] Fig. 13 shows a structural drawing of HER2-V2122.
[Fig. 14] Fig. 14 shows a CBB-stained SDS-PAGE electrophoretic pattern of HER2-V2122.
[Fig. 15] Fig. 15 shows evaluation of the binding performance of HER2-Cupid with an antigen (HER2).
[Fig. 16] Fig. 16 shows evaluation of the binding performance of HER2-Cupid with a modified biotin.
[Fig. 17] Fig. 17 shows *in vitro* cytotoxicity obtained by a combination of HER2-Cupid and a photoactivatable compound-binding modified biotin.

### Embodiment of Carrying out the Invention

Hereinafter, the present invention will be described in more detail.

### < Fusion protein of antibody and mutant streptavidin >

The fusion protein of the present invention is a fusion protein having the amino acid sequence as set forth in SEQ ID NO: 2 or SEQ ID NO: 7, a linker sequence, and the amino acid sequence as set forth in SEQ ID NO: 1 (provided that the amino acid sequence portion consisting of 6 histidine residues at the C-terminus thereof may be partially or entirely deleted), from the N-terminal side to the C-terminal side, in this order. The fusion protein of the present invention may also be a fusion protein having the amino acid sequence of an anti-desmoglein antibody, a linker sequence, and the amino acid sequence as set forth in SEQ ID NO: 1 (provided that the amino acid sequence portion consisting of 6 histidine residues at the C-terminus thereof may be partially or entirely deleted), from the N-terminal side to the C-terminal side, in this order.

The amino acid sequence as set forth in SEQ ID NO: 2 is the amino acid sequence of an scFv-type anti-CEACAM antibody. The amino acid sequence as set forth in SEQ ID NO: 7 is the amino acid sequence of an scFv-type anti-Her2 antibody.

The amino acid sequence as set forth in SEQ ID NO: 1 is the amino acid sequence of a mutant streptavidin, and specifically, this mutant streptavidin is mutant streptavidin LISA314-V2122 described in Example 3 of International Publication WO2015/125820 (SEQ ID NO: 4 of International Publication WO2015/125820) (SEQ ID NO: 1 of the description of the present application).

As described above, the fusion protein of the present invention is a fusion protein of an antibody that recognizes cancer cells and a mutant streptavidin.

The linker sequence is not particularly limited, as long as it can achieve the effects of the present invention. The number of amino acids of the linker sequence is preferably 5 to 15, more preferably 7 to 13, and further preferably 9 to 11.

A specific example of the linker sequence may be a sequence consisting of 4 to 14 glycine residues and 1 cysteine residue. As such a linker sequence, an amino acid sequence represented by, for example, (Gly)ₘ-Cys-(Gly)ₙ (wherein m and n each independently represent an integer of 1 to 13) can be used. Specific example of the linker sequence may include Gly-Gly-Gly-Gly-Ser-Gly-Gly-Gly-Gly and Gly-Gly-Gly-Gly-Gly-Ser-Gly-Gly-Gly-Gly-Gly, but the examples are not particularly limited thereto.

A specific example of the fusion protein of the present invention may be a fusion protein having the amino acid sequence as set forth in SEQ ID NO: 3 or SEQ ID NO: 8 (provided that the amino acid sequence portion consisting of 6 histidine residues at the C-terminus thereof may be partially or entirely deleted).

The fusion protein of the present invention may also have a secretory signal sequence at the N-terminus thereof. The fusion protein having a secretory signal sequence may be a fusion protein having the amino acid sequence as set forth in SEQ ID NO: 4 or SEQ ID NO: 9 (provided that the amino acid sequence portion consisting of 6 histidine residues at the C-terminus thereof may be partially or entirely deleted).

According to the present invention, a nucleic acid (for example, DNA) encoding the above-described fusion protein of the present invention is further provided. A specific example of the nucleic acid of the present invention may be a nucleic acid encoding a fusion protein having the amino acid sequence as set forth in SEQ ID NO: 3 or SEQ ID NO: 8 (provided that the amino acid sequence portion consisting of 6 histidine residues at the C-terminus thereof may be partially or entirely deleted). Another specific example of the nucleic acid of the present invention may be a nucleic acid encoding a fusion protein having the amino acid sequence as set forth in SEQ ID NO: 4 or SEQ ID NO: 9 (provided that the amino acid sequence portion consisting of 6 histidine residues at the C-terminus thereof may be partially or entirely deleted).

A nucleic acid (for example, DNA) encoding the fusion protein of the present invention can be used by being incorporated into a vector. In order to produce the fusion protein of the present invention, a nucleic acid encoding the fusion protein of the present invention is incorporated into an expression vector, and a host is then transformed with this expression vector, so that the fusion protein of the present invention can be expressed in the host.

When *Escherichia coli* is used as a host, the vector preferably has a replication origin (ori) and also has a gene for selecting the transformed host (e.g. a drug-resistance gene that is resistant to drugs, such as ampicillin, tetracycline, kanamycin or chloramphenicol, etc.). Moreover, an expression vector preferably has a promoter capable of efficiently expressing the mutant streptavidin of the present invention in a host, such as a lacZ promoter or a T7 promoter. Examples of such a vector include an M13 vector, a pUC vector, pBR322, pBluescript, pCR-Script, pGEX-5X-1 (Pharmacia), "QIAexpress system" (QIAGEN), pEGFP, and pET (in this case, BL21 that expresses T7 RNA polymerase is preferably used as a host).

A vector can be introduced into a host cell by applying a calcium chloride method or an electroporation method, for example. Further, a sequence that encodes a tag for improving solubility, such as glutathione S-transferase, thioredoxin or a maltose-binding protein, may be added. Still further, a sequence that encodes a tag designed for facilitating purification, such as a polyhistidine tag, a Myc epitope, a hemagglutinin (HA) epitope, a T7 epitope, an Xpress tag, a FLAG tag or other known tag sequences, may also be added.

Other than *Escherichia coli,* examples of the expression vector include: mammal-derived expression vectors (for example, pcDNA3 (manufactured by Invitrogen), pEGF-BOS (Nucleic Acids. Res.1990, 18(17), p. 5322), pEF and pCDM8); insect cell-derived expression vectors (for example, "Bac-to-BAC baculovirus expression system" (manufactured by Gibco-BRL) and pBacPAK8); plant-derived expression vectors (for example, pMH1 and pMH2); animal virus-derived expression vectors (for example, pHSV, pMV and pAdexLcw); retrovirus-derived expression vectors (for example, pZIPneo); yeast-derived expression vectors (for example, "Pichia Expression Kit" (manufactured by Invitrogen), pNV11 and SP-Q01); and *Bacillus subtilis-*derived expression vectors (for example, pPL608 and pKTH50).

When the expression of the present mutant streptavidin in an animal cell such as a CHO cell, a COS cell or an NIH3T3 cell is intended, it is essential for the expression vector to have a promoter necessary for the expression of the mutant streptavidin in such an animal cell, such as an SV40 promoter (Mulligan et al., Nature (1979) 277, 108), an MMLV-LTR promoter, an EF1α promoter (Mizushima et al., Nucleic Acids Res. (1990) 18, 5322) or a CMV promoter. It is more preferable if the expression vector has a gene for selecting the transformation of a cell (for example, a drug-resistance gene capable of determining transformation with the use of drugs (neomycin, G418, etc.)). Examples of a vector having such properties include pMAM, pDR2, pBK-RSV, pBK-CMV, pOPRSV and pOP13.

The type of a host cell, into which the vector is introduced, is not particularly limited. Either prokaryotes or eukaryotes may be used. It is possible to use *Escherichia coli* or various types of animal cells, for example.

In the case of using a eukaryotic cell, for example, an animal cell, a plant cell or a fungal cell can be used as a host. Examples of an animal cell that can be used herein include: mammalian cells such as a CHO cell, a COS cell, a 3T3 cell, a HeLa cell or a Vero cell; and insect cells such as Sf9, Sf21 or Tn5. When the expression of a large amount of the mutant streptavidin in an animal cell is intended, a CHO cell is particularly preferable. A vector can be introduced into a host cell by a calcium phosphate method, a DEAE-dextran method, a method using cationic ribosome DOTAP (manufactured by Boehringer Mannheim), an electroporation method, a lipofection method or the like.

As a plant cell, a cell from *Nicotiana tabacum* has been known as a protein-producing system, for example. These cells may be subjected to callus culture. Examples of a known fungal cell include: yeast cells including genus Saccharomyces such as *Saccharomyces cerevisiae*; and filamentous fungi including genus Aspergillus such as *Aspergillus niger.*

Examples of a procaryotic cell that can be used herein include *Escherichia coli* (*E. coli*), such as JM109, DH5α or HB101. Moreover, *Bacillus subtilis* has been known.

These cells are transformed with the nucleic acid of the present invention, and the transformed cells are then cultured *in vitro,* so as to obtain the fusion protein of the present invention. The culture can be carried out in accordance with a known culture method. Examples of a culture solution of animal cells that can be used herein include DMEM, MEM, RPMI1640, and IMDM. During the culture, a serum infusion such as fetal calf serum (FCS) may be used in combination, or serum free culture may also be carried out. The pH applied during the culture is preferably approximately pH 6 to 8. The culture is generally carried out at a temperature of approximately 30°C to 40°C for approximately 15 to 200 hours. As necessary, medium replacement, ventilation and stirring are carried out. Furthermore, growth factors may also be added to promote the growth of cells.

### < Cancer therapeutic agent or cancer diagnostic agent >

The fusion protein of the present invention is useful as a cancer therapeutic agent or a cancer diagnostic agent.

According to the present invention, provided is a kit for treating or diagnosing cancer, comprising (1) the fusion protein of the present invention, and (2) a conjugate of a compound represented by a formula (1) as shown below or a salt thereof, and a diagnostic substance or a therapeutic substance.

By administering the fusion protein of the present invention to a patient, a mutant streptavidin can be accumulated in the body of the patient, specifically into cancer cells. Subsequently, by administering a conjugate of a biotin-modified dimer having an affinity for the mutant streptavidin and a diagnostic substance or a therapeutic substance to the patient, it becomes possible to accumulate the diagnostic substance or the therapeutic substance precisely into the cancer cells.

Otherwise, a complex is prepared by binding the "fusion protein of the present invention" with the "conjugate of a biotin-modified dimer having an affinity for a mutant streptavidin and a diagnostic substance or a therapeutic substance," and the thus prepared complex can be administered to the patient.

### < Biotin-modified dimer >

The biotin-modified dimer is a compound represented by the following formula (1) or a salt thereof, and is preferably a compound represented by the following formula (2) or a salt thereof. As such a biotin-modified dimer, the compound described in International Publication WO2015/125820 can be used. 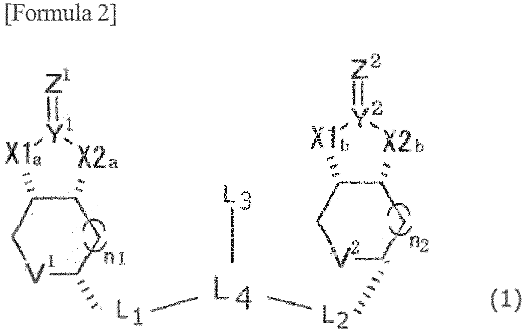 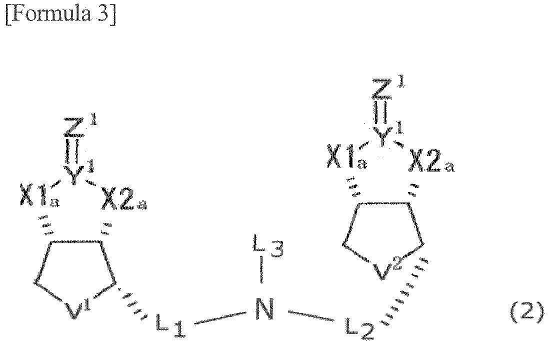 wherein
X1a, X1b, X2a and X2b each independently represent O or NH,
Y¹ and Y² each independently represent C or S,
Z¹ and Z² each independently represent O, S or NH,
V¹ and V² each independently represent S or S⁺-O⁻,
n1 and n2 each independently represent an integer of 0 or 1,
L₁ and L₂ each independently represent a divalent linking group,
L₃ represents a group comprising a functional group capable of binding to the diagnostic substance or the therapeutic substance (for example, a phthalocyanine dye) at the terminus, and L₄ represents a trivalent linking group.

In the formula (1) and the formula (2), the portions represented by the following structures: 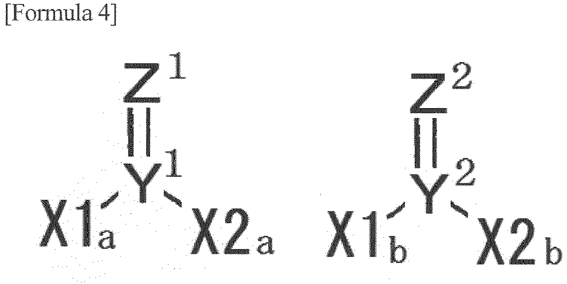 are preferably any one of the following portions, but are not limited thereto:

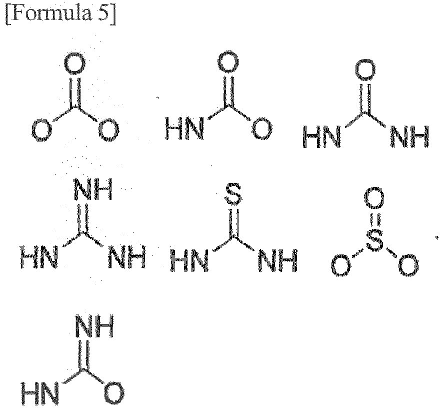

X1a, X1b, X2a and X2b preferably represent NH; Y¹ and Y² preferably represent C; Z¹ and Z² preferably represent NH; and V¹ and V² preferably represent S.

L₁ and L₂ each independently represent a divalent linking group consisting of a combination of groups selected from -CONH-, -NHCO-, -COO-, -OCO-, -CO-, -O-, and an alkylene group containing 1 to 10 carbon atoms.

Preferably, L₁ and L₂ each independently represent a divalent linking group consisting of a combination of groups selected from -CONH-, -NHCO-, -O-, and an alkylene group containing 1 to 10 carbon atoms.

Preferably, L₁ and L₂ each independently represent a divalent linking group consisting of a combination of groups selected from -CONH-, -NHCO-, and an alkylene group containing 1 to 10 carbon atoms.

L₄ represents a trivalent linking group, and is preferably the following: 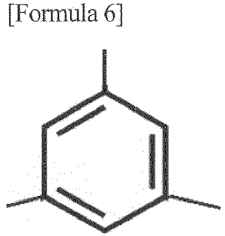 or, 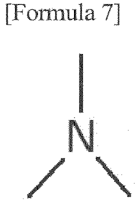 (which is a benzene-derived trivalent linking group or a nitrogen atom).

L₃ is preferably a group consisting of a combination of groups selected from -CONH-, - NHCO-, -COO-, -OCO-, -CO-, -O-, and an alkylene group containing 1 to 10 carbon atoms, and further comprising an amino group at the terminus.

### < Conjugate of biotin-modified dimer and diagnostic substance or therapeutic substance >

By binding a diagnostic substance or a therapeutic substance to a biotin-modified dimer, a conjugate of the biotin-modified dimer and the diagnostic substance or the therapeutic substance can be prepared. Examples of the diagnostic substance or the therapeutic substance may include a fluorochrome, a chemiluminescent agent, a radioisotope, a sensitizer consisting of a metal compound or the like, a neutron-capturing agent consisting of a metal compound or the like, a phthalocyanine dye, a low-molecular-weight compound, micro- or nano-bubbles, and a protein. Preferably, a phthalocyanine dye can be used.

### < Phthalocyanine dye >

The phthalocyanine dye is preferably a silicon phthalocyanine dye. Specific examples of the phthalocyanine dye, such as IRDye (registered trademark) 700DX, are described in, for example, U.S. Patent No. 7,005,518.

As such a phthalocyanine dye, a dye represented by the following formula (21) can be used. As an example, a dye represented by the following formula (22) can be used.

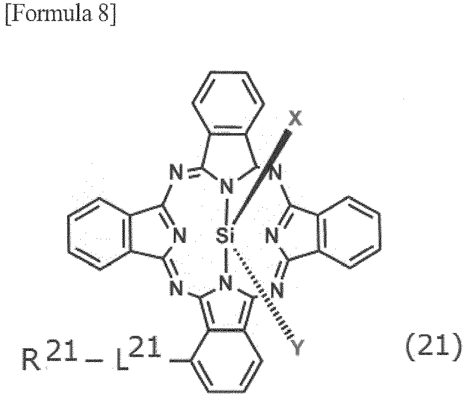

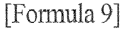

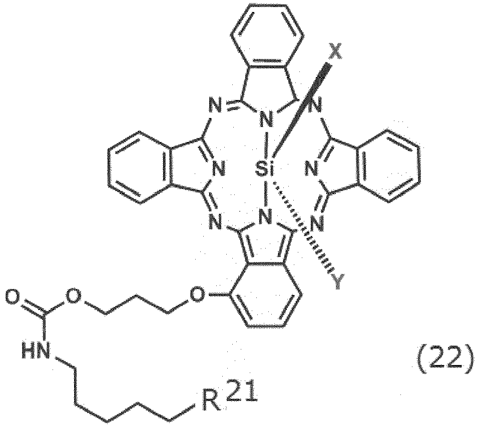

In the above formulae, L²¹ represents a divalent linking group, R²¹ represents a functional group capable of binding to the compound represented by the formula (1) or a salt thereof

In the above formulae, X and Y each independently represent a hydrophilic group, -OH, a hydrogen atom, or a substituent. Examples of the substituent used herein may include, but are not particularly limited to, a halogen atom (a fluorine atom), a substituent containing a carbon atom (a hydrocarbo group, etc.), and a substituent containing a nitrogen atom (an amino group, etc.).

Specific examples of X and Y may include:
(i) a case where both X and Y are hydrophilic groups;
(ii) a case where either X or Y is a hydrophilic group, and the other is -OH or a hydrogen atom; and
(iii) a case where X and Y are -OH or hydrogen atoms.

The hydrophilic group(s) represented by X and/or Y are not particularly limited. One example is shown below.

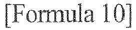

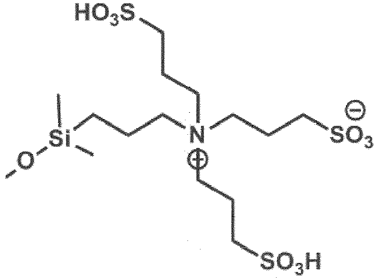

As a phthalocyanine dye, a commercially available product such as IRDye (registered trademark) 700DX can be used. In the present invention, an NHS ester of IRDye (registered trademark) 700DX is used, and is allowed to react with a biotin-modified dimer having an amino group to produce a conjugate. Other variations of IRDye (registered trademark) 700DX are described in U.S. Patent No. 7,005,518, and those can also be used.

R²¹ represents a functional group capable of binding to the compound represented by the formula (1) or a salt thereof. R²¹ is preferably a functional group that can react with a carboxyl group, amine or a thiol group on the biotin-modified dimer and can bind thereto. Preferred examples of R²¹ may include, but are not particularly limited to, activated ester, halogenated acyl, halogenated alkyl, appropriately substituted amine, anhydride, carboxylic acid, carbodiimide, hydroxyl, iodoacetamide, isocyanate, isothiocyanate, maleimide, NHS ester, phosphoramidite, sulfonic acid ester, thiol, and thiocyanate.

L²¹ represents a divalent linking group, and for example, it may include: any given combination of an ether, thioether, amine, ester, carbamate, urea, thiourea, oxy or amide bond, or a single, double, triple or aromatic carbon-carbon bond; or a phosphorus-oxygen, phosphorus-sulfur, nitrogen-nitrogen, nitrogen-oxygen, or nitrogen-platinum bond; or an aromatic or heteroaromatic bond. L²¹ is preferably a group consisting of a combination of groups selected from -CONH-, - NHCO-, -COO-, -OCO-, -CO-, -O-, and an alkylene group containing 1 to 10 carbon atoms.

-L²¹-R²¹ may include a phosphoramidite group, NHS ester, active carboxylic acid, thiocyanate, isothiocyanate, maleimide, and iodoacetamide.

L²¹ represents a -(CH₂)ₙ- group, and in the formula, n is an integer of 1 to 10, and is preferably an integer of 1 to 4. As one example, -L²¹-R²¹ is -O-(CH₂)₃-OC(O)-NH-(CH₂)₅-C(O)ON-succinimidyl.

### < Photoimmunotherapy >

Photoimmunotherapy is a therapeutic method of using a photosensitizer and an irradiation light to destroy specific cells in a body. When a photosensitizer is exposed to a light with a specific wavelength, it generates cytotoxic reactive oxygen species capable of inducing apoptosis, necrosis, and/or autophagy to around cells. For example, Japanese Patent No. 6127045 discloses a method of killing cells, comprising: a step of allowing cells comprising a cell surface protein to come into contact with a therapeutically effective amount of one or more antibodies-IR700 molecules, wherein the antibodies specifically bind to the cell surface protein; a step of irradiating the cells with a light at a wavelength of 660 to 740 nm and at a dose of at least 1 Jcm⁻²; and a step of allowing the cells to come into contact with one or more therapeutic agents at approximately 0 to 8 hours after the irradiation, thereby killing the cells. JP Patent Publication (Kohyo) No. 2017-524659 A discloses a method of inducing cytotoxicity to a subject affected with a disease or a pathology, comprising: (a) administering to a subject, a therapeutically effective drug comprising a phthalocyanine dye such as IRDye (registered trademark) 700DX conjugated with a probe specifically binding to the cell of the subject; and (b) irradiating the cell with an appropriate excitation light in an amount effective for inducing cell death.

The fusion protein of the present invention and the conjugate of a biotin-modified dimer and a phthalocyanine dye are administered to a subject, and the cells are then irradiated with an excitation light in an amount effective for suppression of cell proliferation or induction of cell death, so that the cell proliferation can be suppressed or the cell death can be induced, and thereby the subject can be treated.

Preferably, the fusion protein of the present invention and the conjugate of a biotin-modified dimer and a phthalocyanine dye are administered to a subject, and the cells are then irradiated with an excitation light in an amount effective for suppression of cell proliferation or induction of cell death, so that the cell proliferation can be suppressed or the cell death can be induced, and thereby the subject can be treated.

The subject used herein includes humans and non-human animals. Examples of the subject may include humans and experimental animals such as mice. The subject is preferably affected with a disease regarding which suppression of cell proliferation or induction of cell death is desired. For example, the subject is affected with cancer or solid tumor.

Examples of the "cancer" may include carcinoma, lymphoma, blastoma, sarcoma, and leukemia or malignant lymphoma. Specific examples of the cancer may include squamous cell carcinoma (e.g., epithelial squamous cell carcinoma), lung cancer including small cell lung cancer, non-small cell lung cancer ("NSCLC"), pulmonary adenocarcinoma and pulmonary squamous cell carcinoma, peritoneal cancer, hepatocarcinoma, corpus ventriculi or stomach cancer, including digestive cancer, pancreatic cancer, glioblastoma, cervical cancer, ovarian cancer, liver cancer, bladder cancer, hepatocellular cancer, breast cancer, colon cancer, rectal cancer, colorectal cancer, endometrial membrane cancer or endometrial carcinoma, salivary gland carcinoma, kidney or renal region cancer, prostate cancer, vulvar cancer, thyroid cancer, hepatocellular carcinoma, anal carcinoma, penile carcinoma, and head and neck cancer.

The solid tumor means a benign or malignant, abnormal cell mass that generally does not contain a capsule. Examples of the solid tumor may include glioma, astrocytoma, medulloblastoma, craniopharyngioma, ependymoma, pineal gland tumor, hemangioblastoma, acoustic neuroma, oligodendrocyte, meningioma, melanoma, neuroblastoma, and retinoblastoma.

Examples of the administration method to the subject may include, but are not limited to, a local route, an injection (a subcutaneous injection, an intramuscular injection, an intradermal injection, an intraperitoneal injection, an intratumoral injection, an intravenous injection, etc.), an oral route, an ocular route, a sublingual route, a rectal route, a percutaneous route, an intranasal route, a vaginal route, and an inhalation route.

It is preferable that the conjugate of a biotin-modified dimer and a phthalocyanine dye and the fusion protein of the present invention are each administered in a therapeutically effective amount. Regarding each of the above-described conjugate and fusion protein, the therapeutically effective amount per 60 kg is at least 0.5 mg (mg/60 kg), at least 5 mg/60 kg, at least 10 mg/60 kg, at least 20 mg/60 kg, at least 30 mg/60 kg, or at least 50 mg/60 kg. For example, when it is intravenously administered, the applied dose is 1 mg/60 kg, 2 mg/60 kg, 5 mg/60 kg, 20 mg/60 kg, or 50 mg/60 kg, and it is, for example, 0.5 to 50 mg/60 kg. In another example, the therapeutically effective amount is at least 100 µg/kg, at least 500 µg/kg or at least 500 µg/kg, and it is, for example, at least 10 µg/kg. For example, when it is intratumorally or intraperitoneally administered, the dose is 100 µg/kg, 250 µg/kg, approximately 500 µg/kg, 750 µg/kg, or 1000 µg/kg, and it is, for example, 10 µg/kg to 1000 µg/kg. In one example, when it is administered in the form of a solution for local administration, the therapeutically effective amount is 10 µg/ml, 20 µg/ml, 30 µg/ml, 40 µg/ml, 50 µg/ml, 60 µg/ml, 70 µg/ml, 80 µg/ml, 90 µg/ml, 100 µg/ml or the like, or it is 20 µg/ml to 100 µg/ml, or it is at least 500 µg/ml, or at least 1 µg/ml.

The above-described dose can be administered once or divided doses over several administrations (2, 3, or 4 times, etc.), or as a single preparation.

The conjugate of a biotin-modified dimer and a phthalocyanine dye and the fusion protein of the present invention can be each administered alone, or can also be administered in the presence of a pharmaceutically acceptable carrier, or can also be administered in the presence of other therapeutic agents (other anticancer agents, etc.).

The conjugate of a biotin-modified dimer and a phthalocyanine dye and the fusion protein of the present invention can bind to target cells or target tissues, such as circulating tumor cells or solid tumor cells. Thereafter, the target cells or tissues are irradiated with a light, so that the above-described conjugate or complex can absorb the light and can damage or destroy the target cells or tissues.

In the photoimmunotherapy, the wavelength of the irradiation light is preferably 660 to 740 nm, and the irradiation light has a wavelength of, for example, 660 nm, 670 nm, 680 nm, 690 nm, 700 nm, 710 nm, 720 nm, 730 nm, or 740 nm. Light irradiation may be carried out using a device equipped with a near infrared (NIR) light emitting diode.

The light irradiation amount is at least 1 J/cm², for example, at least 4 J/cm², at least 10 J/cm², at least 15 J/cm², at least 20 J/cm², at least 50 J/cm², or at least 100 J/cm². It is, for example, 1 to 500 J/cm². Light irradiation may be carried out several times (e.g., 2, 3,4, 5, 6, 7, 8, 9, or 10 times).

The present invention will be more specifically described in the following examples. However, these examples are not intended to limit the scope of the present invention.

### Examples

### < Production Example 1 >

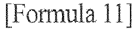

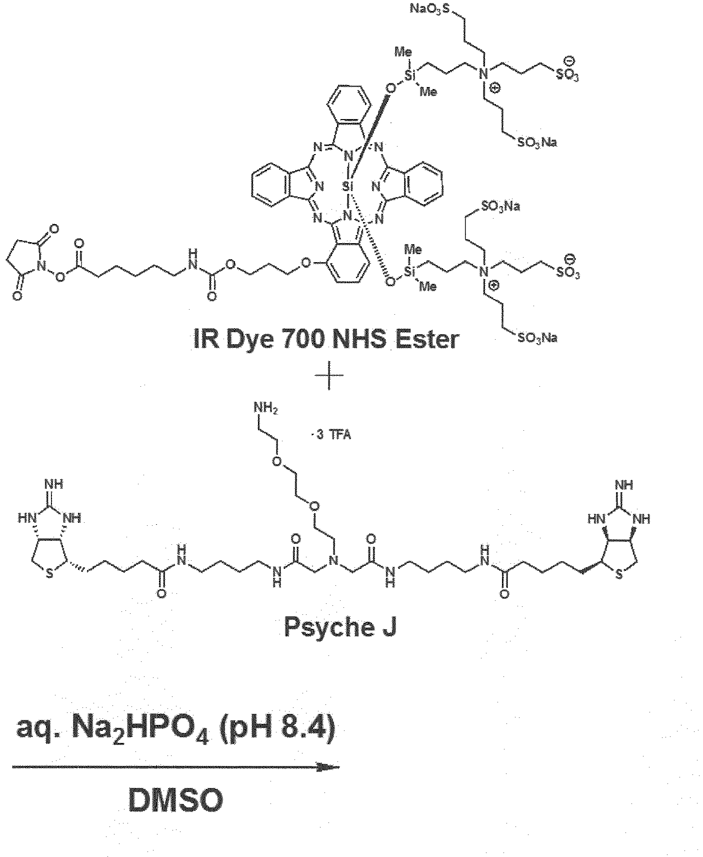

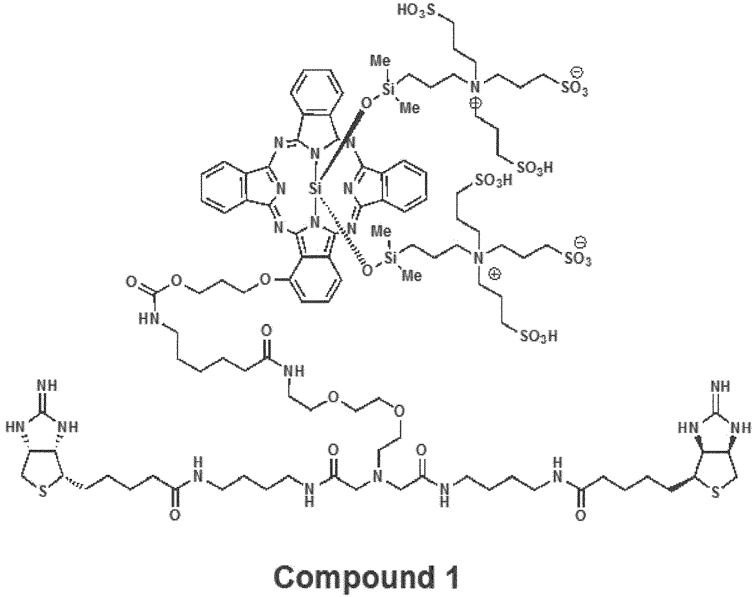

IR Dye 700 NHS Ester (3.0 mg, 1.5 µmol), a disodium hydrogen phosphate buffer (pH 8.4, 144 µL) and dimethyl sulfoxide (120 µL) were added to Psyche J (1.8 mg, 1.6 µmοl), light was then shielded with an aluminum foil, and the mixture was then stirred at room temperature for 24 hours. Thereafter, the reaction solution diluted with water to 500 µL was purified by reverse phase HPLC (gradient: 0% for 5 min; 0-100% for 100 min acetonitrile in a 50 mM triethylammonium acetate aqueous solution (pH 6.5), retention time = 47.2 min, YMC-Triart C18, flow rate = 3.5 mL/min) to obtain a target compound 1 (dark blue green). Quantification was carried out from the reported molar absorption coefficient of IR700 at 694 nm in water (165,000 (M⁻¹ cm⁻¹)), and the yield (1.0 µmol, 72%) was calculated.
LRMS (ESI): *m*/*z* 1303 [M+2H]²⁺, 869 [M+3H]³⁺

### < Production Example 2 >

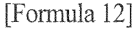

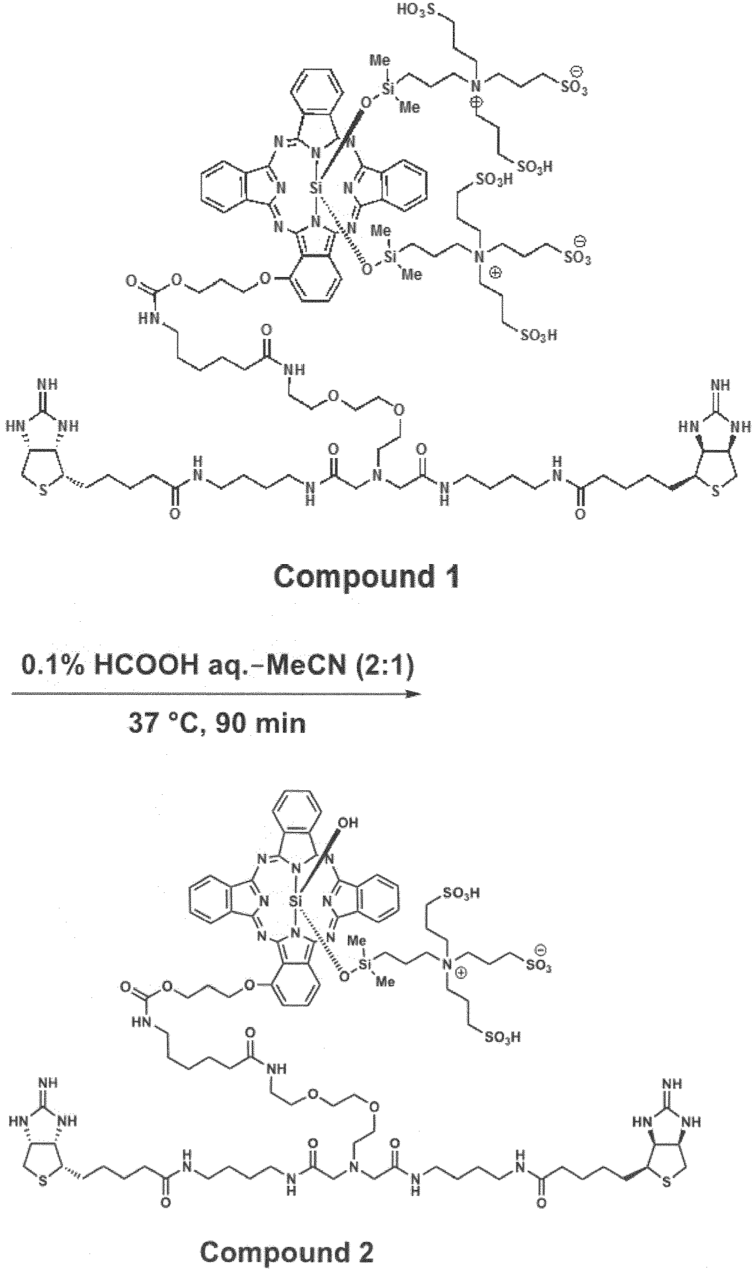

A 0.1% formic acid aqueous solution (266 µL) and acetonitrile (133 µL) were added to the compound 1 (0.375 mg, 144 nmol), and the obtained mixture was stirred with a Vortex mixer and was then centrifuged. The resultant was left at rest under conditions of in a darkroom at 37°C for 90 minutes. The resulting solution was purified by reverse phase HPLC (gradient: 0% for 5 min; 0-100% for 100 min acetonitrile in a 50 mM triethylammonium acetate aqueous solution (pH 6.5), retention time = 51.6 min, YMC-Triart C18, flow rate = 3.5 mL/min) to obtain a target compound 2 (dark blue green). Quantification was carried out in the same manner as described above, and the yield (48 nmol, 33%) was calculated.
LRMS (ESI): *m*/*z* 1054 [M-H₂O+2H]²⁺, 703 [M-H₂O+3H]³⁺

### < Production Example 3 >

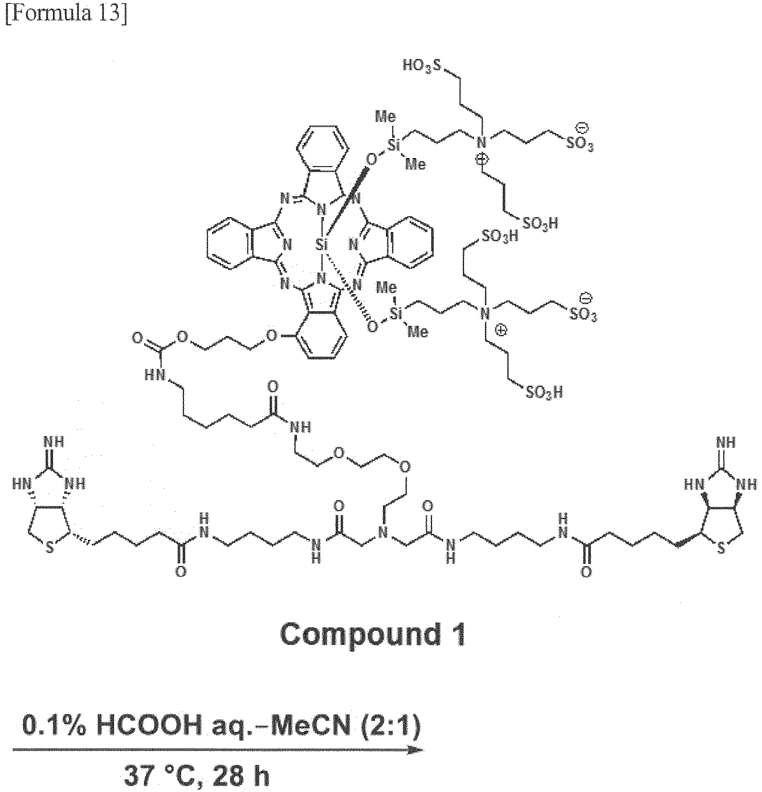

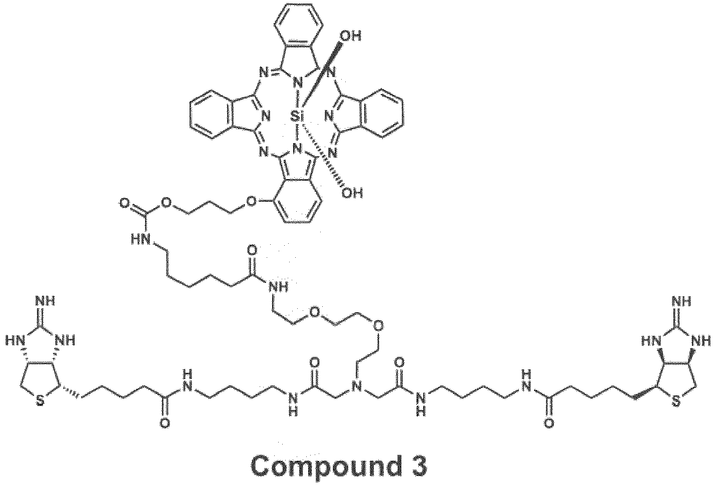

A 0.1% formic acid aqueous solution (266 µL) and acetonitrile (133 µL) were added to the compound 1 (0.375 mg, 144 nmol), and the obtained mixture was stirred with a Vortex mixer and was then centrifuged. The resultant was left at rest under conditions of in a darkroom at 37°C for 28 hours. The resulting solution was purified by reverse phase HPLC (gradient: 0% for 5 min; 0-100% for 100 min acetonitrile in a 50 mM triethylammonium acetate aqueous solution (pH 6.5), retention time = 56.2 min, YMC-Triart C18, flow rate = 3.5 mL/min) to obtain a target compound 3 (dark blue green). Quantification was carried out in the same manner as described above, and the yield (15 nmol, 10%) was calculated.
LRMS (ESI): *m*/*z* 813 [M-H₂O+2H]²⁺, 542 [M-H₂O+3H]³⁺

### Example 1: Expression and purification of CEA-V2122 protein

V2122 is the mutant streptavidin described in Example 3 of International Publication WO2015/125820 (SEQ ID NO: 4 shown in International Publication WO2015/125820). The amino acid sequence of V2122 (a sequence having a 6xHis tag at the C-terminus) is as set forth in SEQ ID NO: 1 in the sequence listing.

scFv-V2122 is prepared by binding a single-chain antibody (scFv) against CEACAM5 with the above-described V2122. This scFv-type anti-CEACAM5 antibody is an scFv sequence described in a patent document US7626011B2. The amino acid sequence of the scFv-type anti-CEACAM5 antibody is as set forth in SEQ ID NO: 2 in the sequence listing. In addition, the amino acid sequence of CEA-V2122 prepared by binding the scFv-type anti-CEACAM5 antibody with V2122 via an amino acid linker (GGGGSGGGG) (SEQ ID NO: 11) is as set forth in SEQ ID NO: 3 in the sequence listing.

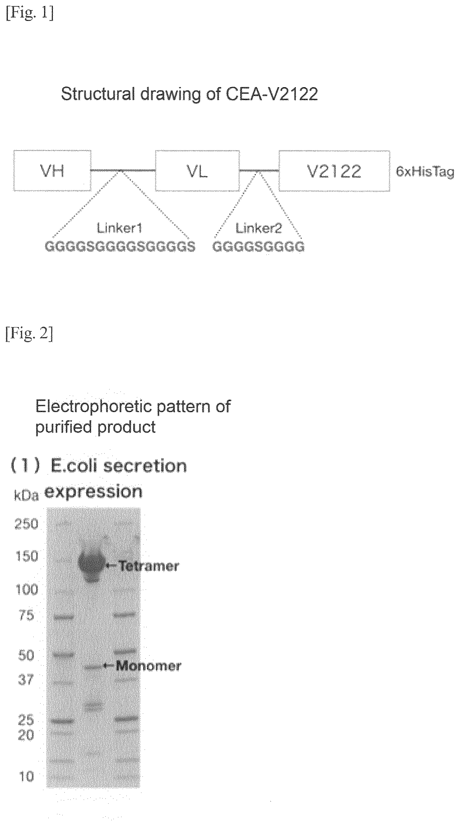

For the expression of a CEA-V2122 fusion protein, the DNA codon of a CEA-V2122 gene sequence, in which a pelB signal for secretion and expression in *Escherichia coli* had been incorporated into the N-terminus and a 6xHis-Tag sequence had been incorporated into the C-terminus, was optimized for *Escherichia coli,* thereby synthesizing an artificial gene. This amino acid sequence is as set forth in SEQ ID NO: 4 in the sequence listing, and the DNA sequence is as set forth in SEQ ID NO: 5 in the sequence listing. Moreover, an outline of a domain structure is shown in Fig. 1.

As a specific protein expression vector, a vector prepared by incorporating a chaperone skp gene into MCS2 of a pETOuet1 vector was used. Regarding the skp gene, the DNA codon was optimized for *Escherichia coli* based on the amino acid sequence as set forth in SEQ ID NO: 6 in the sequence listing, thereby synthesizing an artificial gene. The synthesized skp gene was amplified by PCR, using the primers (AAGGAGATATACATATGGATAAAATTGCCATTGTTAATAT (SEQ ID NO: 12), and TTGAGATCTGCCATATGTTATTTCACTTGTTTCAGAACG (SEQ ID NO: 13)), and the amplified gene was then cloned into MCS2 of the pETDue1 vector linearized with the restriction enzyme NdeI, using In-Fusion HD Cloning Kit, so as to obtain pETDuet_skp. Subsequently, the CEA-V2122 gene was incorporated into MCS1 of pETDuct_skp. Specifically, the artificially synthesized CEA-V2122 gene was amplified by PCR, using the primers (AGAAGGAGATATACCATGAAATATCTGCTGCCGAC (SEQ ID NO: 14), and CGCCGAGCTCGAATTTTAATGATGGTGATGATGATG (SEQ ID NO: 15)). Moreover, pETDuet_skp was linearized by PCR, using the primers (GGTATATCTCCTTCTTAAAGTTAAAC (SEQ ID NO: 16), and AATTCGAGCTCGGCGCGCCTGCAG (SEQ ID NO: 17)). The CEA-V2122 amplified by PCR and the linearized pETDuet_skp were subjected to cloning using In-Fusion HD Cloning Kit. The cloned vector was confirmed by sequencing, in terms of the gene sequence incorporated therein, and thereafter, it was referred to as pETDuet_CEA-V2122_skp.

For the expression of the protein, pETDuet_CEA-V2122_skp was transformed into BL21(DE3) (Nippon Gene Co., Ltd.), which was then pre-cultured in 2xYT medium (SIGMA-ADLRICH) at 37°C overnight. The medium used in the pre-culture was added to a new medium for 100-fold dilution, and culture was then carried out at 37°C, until OD (600 nm) became 0.5 to 2.0. Subsequently, IPTG was added to the culture to a final concentration of 0.5 mM, and the obtained mixture was then cultured at 37°C for 4 hours. Thereafter, a culture supernatant was recovered and was then preserved at 4°C.

The CEA-V2122 protein was roughly purified according to a batch method utilizing 6xHis-Tag added to the C-terminus. Specifically, cOmplete His-Tag Purification Resin equilibrated with buffer A (50 mM Tris-HCl, 0.2 M NaCl, 1 mM EDTA, and 5 mM Imidazole; pH 8.0) was added to the culture supernatant preserved at 4°C. The obtained mixture was stirred for 2 hours to overnight at 4° C, so that the protein was allowed to bind to the resin. Subsequently, the resin was recovered into a column, and a 20 column volume of washing operation was performed with buffer A. Thereafter, a roughly purified product of CEA-V2122 was recovered by elution with buffer B (50 mM Tris-HCl, 0.2 M NaCl, 1 mM EDTA, and 400 mM Imidazole; pH 8.0).

Subsequently, the roughly purified product was purified using a Protein L column. Specifically, 1 mL of Capto L (GE Healthcare Life Sciences) was filled into a PD-10 column, and was then equilibrated with 10 column volume of PBS, and the aforementioned roughly purified product was then applied thereto. Thereafter, the resultant was washed with 10 column volume of PBS, was then eluted with 10 mM glycine hydrochloride (pH 2.0), and was then subjected to centrifugal concentration using Vivaspin Turbo 15 (MWCO 100,000). Moreover, using PD-10 (GE Healthcare Life Science), the buffer was replaced with PBS, and centrifugal concentration was further carried out using Vivaspin Turbo 4 (MWCO 100,000) to obtain a finally purified product. After completion of SDS-PAGE electrophoresis, the purity of tetramer CEA-V2122 was assayed by CBB staining. The results are shown in Fig. 2. As an SDS-PAGE gel, Mini-PROTEAN TGX 4-15% (Bio-Rad) was used, and as a CBB staining solution, Bullet CBB Stain One (Ready To Use) (Nacalai Tesque, Inc.) was used.

From Fig. 2, it was confirmed that the purified CEA-V2122 comprises an approximately 150 kDa tetramer as a main component.

### Example 2: Evaluation of performance of CEA-V2122 by SPR

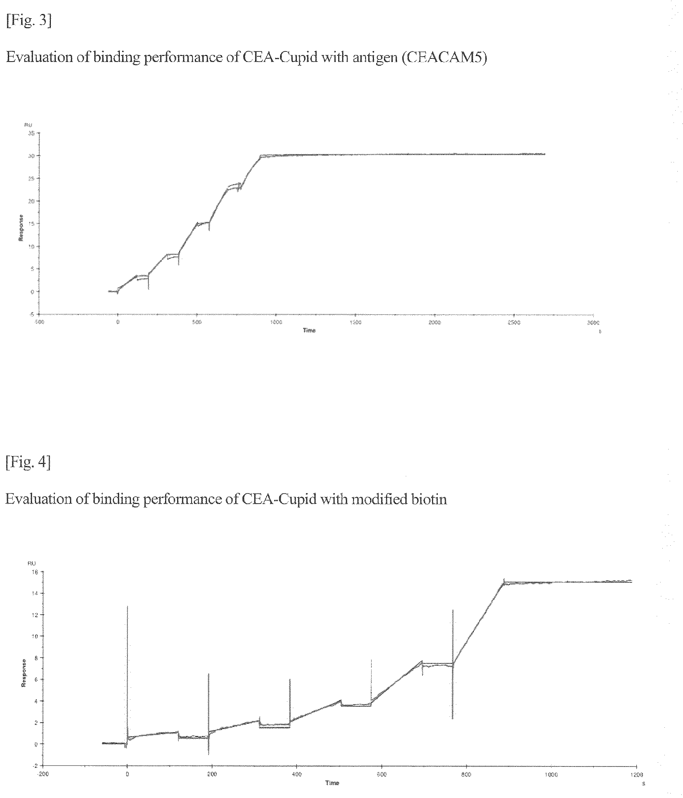

The affinity of CEA-V2122 for the antigen CEACAM5 was evaluated using a surface plasmon resonance (SPR) measuring device, Biacore T200 (GE Healthcare Life Sciences). Specifically, Recombinant Human CEACAM-5/CD66e Protein, CF (R & D SYSTEMS) was immobilized on Sensor Chip CM5 (GE Healthcare Life Sciences) using an amine-coupling kit (GE Healthcare Life Sciences). The final amount of the ligand immobilized was 279 RU. Moreover, with regard to the purified CEA-V2122, two-fold serial dilutions from 1E-08 M to 6.25E-10 M were prepared as analytes. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.208E + 5, and kd1 = 3.461E - 7. Moreover, since evaluation can be carried out at K_{D} = kd1/ka1 in the bivalent analysis, the evaluation value K_{D} = kd1/ka1 = 3.461E - 7 / 3.208E + 5 = 1.078E - 12 was obtained. These results are shown in Fig. 3.

From the K_{D} value in the sensorgram shown in Fig. 3, it was confirmed that CEA-V2122 strongly binds to CEACAM5.

Furthermore, the interaction between CEA-V2122 and a modified biotin was also analyzed using Biacore T200. The modified biotin was specifically the title compound 14 described in Example 1 of International Publication WO2018/07239. In addition, the analysis method was specifically as follows. That is, an amine-coupling kit was used, a target value was set to be 5000 RU, and the purified CEA-V2122 was immobilized on Sensor Chip CM5. With regard to the concentrations of the analytes, 5 types of two-fold serial dilutions from 1E-08 M to 6.25E-10 M were used. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.792E + 4, and kd1 = 4.424E - 6. Moreover, since evaluation can be carried out at K_{D} = kd1/ka1 in the bivalent analysis, the evaluation value K_{D} = kd1/ka1 = 3.792E + 4/4.424E - 6 = 1.167E - 10 was obtained. These results are shown in Fig. 4.

From the K_{D} value in the sensorgram shown in Fig. 4, it was confirmed that CEA-V2122 strongly binds to a modified biotin.

### Example 3: Cell staining of CEACAM5-expressing cell line using FITC-labeled CEA-V2122

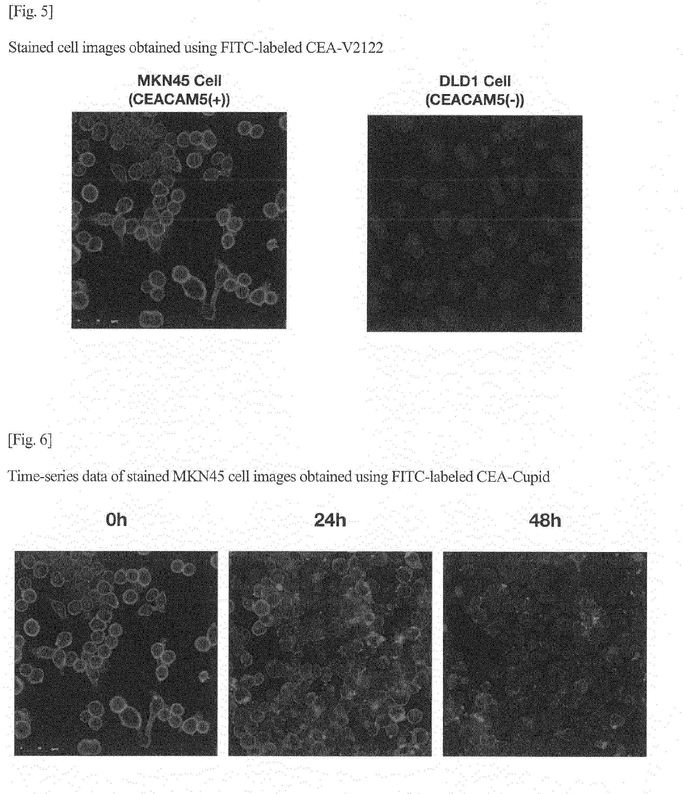

In order to stain a CEACAM5 expression-positive cancer cell line, FITC labeling was carried out on the cell line, using 100 µg of a purified CEA-V2122 protein. Specifically, labeling was carried out using Fluorescein Labeling Kit - NH₂ (DOJINDO LABORATORIES) in accordance with the dosage and administration described in the operating manual included with the kit, and the obtained product was defined to be CEA-V2122-FITC. Specific staining of the CEACAM5 expression-positive cancer cell line is as follows. That is, CEACAM5-positive human stomach cancer-derived MKN-45 cells and CEACAM5-negative human colon cancer-derived DLD1 cells were each seeded on a CELLSTAR, µClear, 96-well plate (Greiner) to a cell density of 2.0 x 10⁴ cells/well, and thereafter, the cells were cultured overnight. Subsequently, a culture solution containing 20 nM CEA-V2122-FITC and 1 µM Hoechist was added to the 96-well plate to a concentration of 100 µL/well, and the obtained mixture was then reacted at 4°C for 30 minutes. Thereafter, each image was taken using In Cell Analyzer 6000 (GE Healthcare Life Sciences). The results are shown in Fig. 5 and Fig. 6.

From the results shown in Fig. 5, it was confirmed that CEA-V2122-FITC specifically recognizes CEACAM5 on the surface of the cell membrane. In addition, from the results shown in Fig. 6, it was confirmed that after CEA-V2122-FITC has bound to CEACAM5, the CEACAM5 stays on the surface of the cell membrane.

### Example 4: In vitro cytotoxicity test using CEA-V2122 and photoactivatable compound-labeled modified biotins

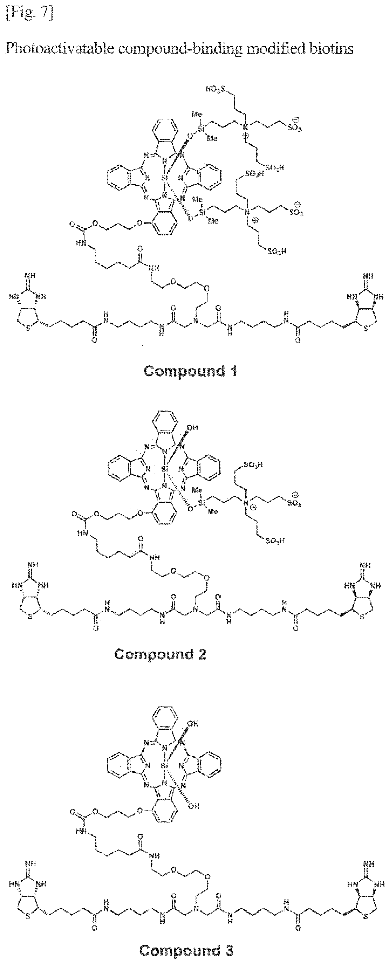

A cytotoxicity test was carried out using the photoactivatable compound-labeled modified biotins, namely, Compound 1, Compound 2, and Compound 3. These compounds are described in Japanese Patent Application No. 2018-149295. Compound 1, Compound 2, and Compound 3 are shown in Fig. 7. Specifically, MKN45 cells were seeded on a 96-well plate for cell culture, so that the cell count became 5 x 10³ cells/well and the amount of the culture solution became 50 µL/well, and thereafter, the cells were cultured overnight. A solution containing a complex of CEA-V2122 and a photoactivatable compound-labeled modified biotin was prepared, so that the molar ratio of CEA-V2122 and each compound became 1 : 2, and the solution was then incubated at room temperature for 10 minutes. Thereafter, the concentration of the reaction solution was adjusted with a culture solution, so that the final concentration of CEA-V2122 became 10 µg/mL. Regarding serial dilutions, 20 µg/mL was set to be an initial concentration, from 4-fold serial dilutions (5.0 µg/mL, 1.25 µg/mL, 0.312 µg/mL, and 0.078 µg/mL), 5 complex serial dilution solutions were prepared. Besides, a medium alone that contained no complex was used as a zero control.

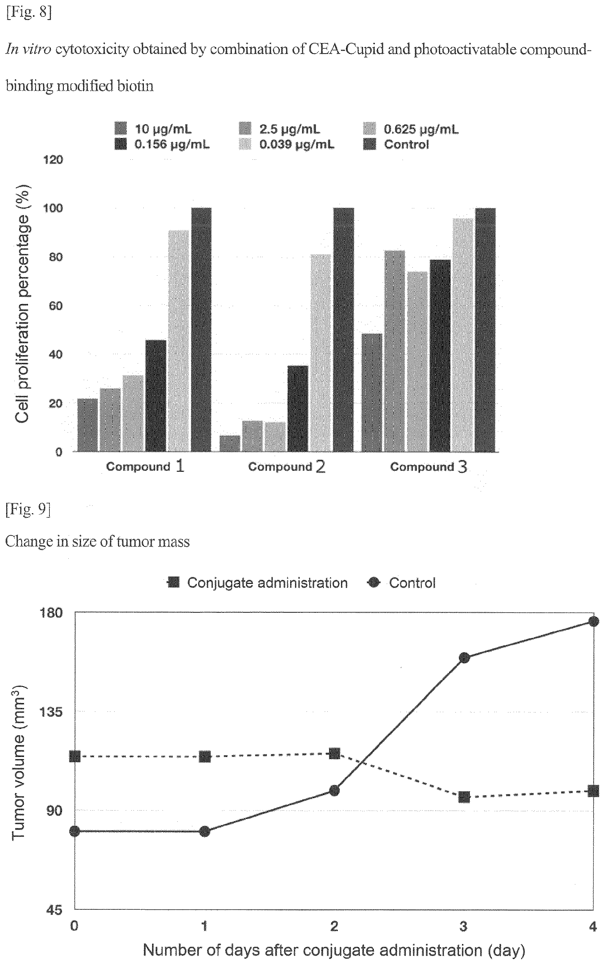

Such complex serial dilution solutions were each added in an amount of 50 µL/well to the cells cultured overnight, so that the final concentrations became 10 µg/mL, 2.5 µg/mL, 0.625 µg/mL, 0.156 µg/mL, and 0.039 µg/mL One hour and two hours after addition of the complex, the cells were irradiated with a light, using LED emitting a light having a wavelength of 690 ± 10 nm, so that the irradiation energy became 100 J/cm². Thereafter, the cells were cultured for 48 hours, and thereafter, a comparison was made in terms of the number of surviving cells, using Cell Counting Kit-8 (DOJINDO LABORATORIES). Each condition was set to be n = 3. The dosage and administration were determined in accordance with the instruction manuals included with the kit, and after addition of the reagent, the mixture was incubated for 1.5 hours at 37°C, in a CO₂ incubator. Thereafter, the absorbance at 450nm was measured, and the mean value was then calculated, followed by background collection. The control was set to be 100%, and the ratio of cell proliferation to the control under each condition was calculated. The results are shown in Fig. 8.

As shown in Fig. 8, it was confirmed that the complex of CEA-V2122, and Compound 1, Compound 2 or Compound 3 exhibits cytotoxicity in a concentration-dependent manner.

### Example 5: In vivo cytotoxicity test using CEA-V2122 and photoactivatable compound-labeled modified biotin, in which xenograft mouse models are employed

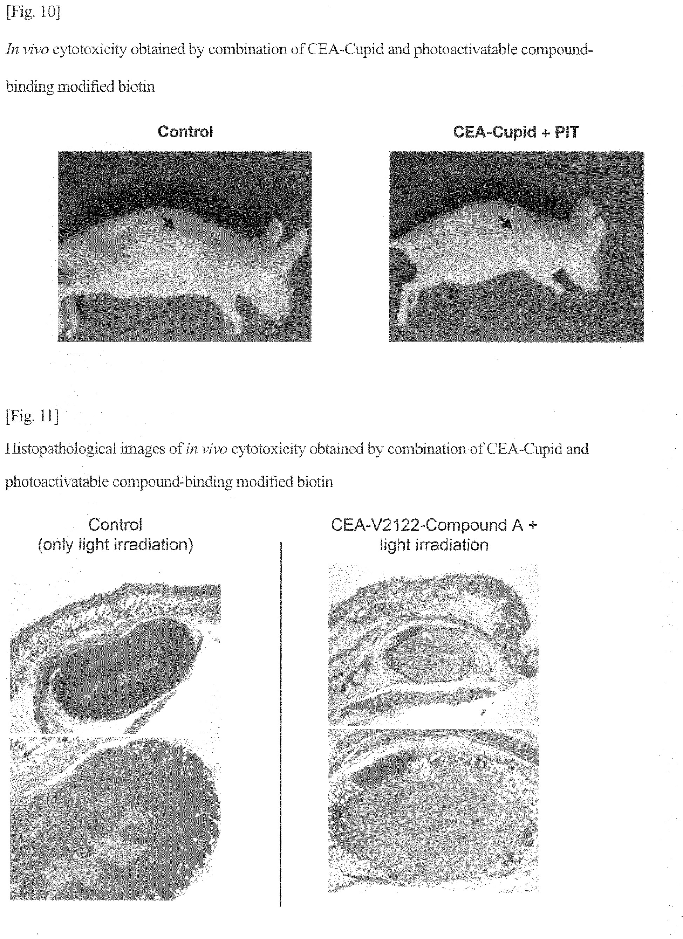

MKN45 cells were subcutaneously transplanted into nude mice to produce xenograft mouse models. That is, 4-week-old female nude mice were purchased, and the mice were then acclimated for 1 week. Thereafter, the MKN45 cells were transplanted in a cell count of 2 x 10⁵ cells per mouse into the subcutis thereof. Approximately 10 days after completion of the transplantation, 100 µg of a complex of CEA-V2122 and a photoactivatable compound-labeled modified biotin (Compound 1), as described in Experiment Example 4, was administered to the mice via the caudal vein thereof. Using an LED light source emitting a light having a wavelength of 690 nm (USHIO OPTO SMICONDUCTORS, INC., LD690D-66-60-550.), T-Cube LED Driver (THORLABS, NC0713145), and T-CUBE 15V POWER SUPPLY (THORLABS, TPS001), 6 hours after the administration, the mice were irradiated with the light having a wavelength of 690 nm, so that the irradiation energy became 230 J/cm². Twenty-four hours after administration of the complex, the mice were irradiated again with the light having a wavelength of 690 nm, so that the irradiation energy became 230 J/cm². A graph showing a change in the tumor volume is shown in Fig. 9. Photographs of the mice 5 days after administration of the complex are shown in Fig. 10. Individual mice were euthanized on the 7th day after administration of the complex and were then excised, and the tumor portions were subjected to pathological analysis. The specific method is as follows. That is, the subcutaneous tumor of each mouse and the peripheral tissues thereof were excised as a mass, and the mass was then immersed in a 4% parafonnaldehyde solution (Wako Pure Chemical Industries, Ltd., 163-20145) at room temperature overnight, so that the tissues were fixed. The thus fixed subcutaneous tumor tissues were divided to a thickness of approximately 3 to 5 mm, so as to produce paraffin-embedded blocks. After that, using a microtome, sliced pathological specimens each having a thickness of 4 µm were produced.

Regarding hematoxylin and eosin staining, the sliced pathological specimen was immersed in a xylene solution (Wako Pure Chemical Industries, Ltd., 241-00091) at room temperature for 10 minutes to perform deparaffinization, and thereafter, hematoxylin staining (Sakura Finetek Japan Co., Ltd., #8650) and eosin staining (Sakura Finetek Japan Co., Ltd., #8660) were carried out. The results are shown in Fig. 11.

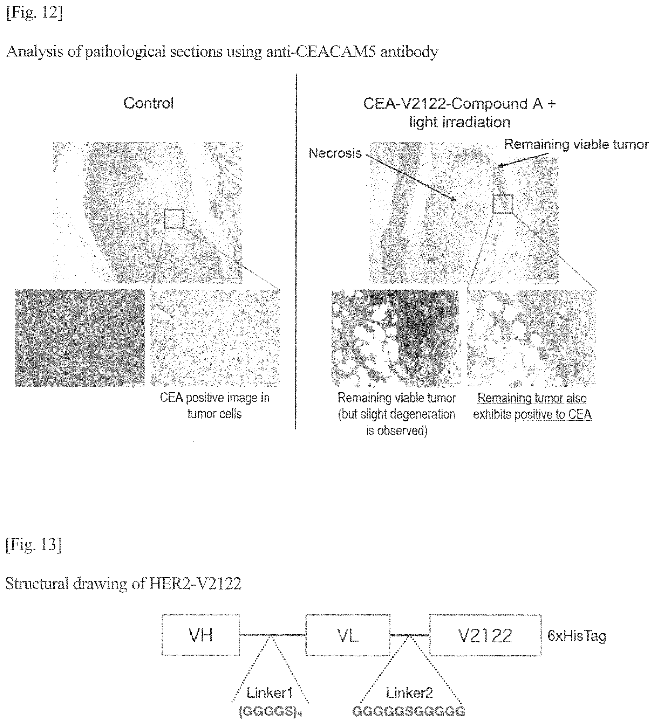

Moreover, immunostaining on CEACAM5 was carried out as follows. The sliced pathological specimen was immersed in a xylene solution (Wako Pure Chemical Industries, Ltd., 241-00091) at room temperature for 10 minutes to perform deparaffinization, and thereafter, the antigen was activated by performing an autoclave treatment (121°C, 5 minutes) using a citrate buffer (pH = 6.0). Thereafter, the resulting specimen was immersed in a 0.3% hydrogen peroxide (Wako Pure Chemical Industries, Ltd., 081-04215)/methanol (Wako Pure Chemical Industries, Ltd., 137-01823) solution at room temperature for 10 minutes to remove endogenous peroxidase. Non-specific reactions were blocked with a 2% BSA (Sigma Aldrich, A1470)/phosphate buffered saline solution. An anti-CEACAM5 antibody (R & D SYSTEMS, MAB41281, concentration: 1/100) was reacted with the specimen at 4°C overnight, and thereafter, immunostaining signals were visualized using Histostar (trademark) (MBL, #8460) and DAB Substrate Solution (MBL, #8469). Finally, nuclear staining was carried out using hematoxylin (Sakura Finetek Japan Co., Ltd., #8650). The results are shown in Fig. 12.

From Fig. 11 and Fig. 12, it was confirmed that a complex of CEA-V2122 and Compound 1 is administered to a xenograft mouse model and the mouse model is then irradiated with a light with a wavelength of 690 nm, so that necrosis can be induced to the tumor of the xenograft mouse model.

### Example 6: Expression and purification of HER2-V2122 protein

V2122 is a mutant streptavidin described in Example 3 of International Publication WO2015/125820 (SEQ ID NO: 4 shown in International Publication WO2015/125820). The amino acid sequence of V2122 is as set forth in SEQ ID NO: 1 in the sequence listing.

scFv-V2122 is prepared by binding a single-chain antibody (scFv) against HER2 (ERBB2) with the above-described V2122. This scFv-type anti-HER2 antibody is an scFv sequence described in Zhang H, et al., Therapeutic potential of an anti-HER2 single chain antibody-DM1 conjugates for the treatment of HER2-positive cancer. Signal Transduct Target Ther. 2017 May 19; 2: 17015. doi: 10.1038/sigtrans. 2017.15. The amino acid sequence of the scFv-type anti-HER2 antibody is as set forth in SEQ ID NO: 7 in the sequence listing. In addition, the structural drawing of HER2-V2122 prepared by binding the scFv-type anti-HER2 antibody with V2122 via an amino acid linker (GGGGGSGGGGG) (SEQ ID NO: 18) is shown in Fig. 13, and the amino acid sequence of HER2-V2122 is as set forth in SEQ ID NO: 8 in the sequence listing.

For the expression of a HER2-V2122 fusion protein, the DNA codon of a HER2-V2122 gene sequence, in which a pelB signal for secretion and expression in *Escherichia coli* had been incorporated into the N-terminus and a 6xHis-Tag sequence had been incorporated into the C-terminus, was optimized for *Escherichia coli,* thereby synthesizing an artificial gene. This amino acid sequence is as set forth in SEQ ID NO: 9 in the sequence listing, and the DNA sequence is as set forth in SEQ ID NO: 10 in the sequence listing.

As a specific protein expression vector, a vector prepared by incorporating a chaperone skp gene into MCS2 of a pETDuet1 vector was used. Regarding the skp gene, the DNA codon was optimized for *Escherichia coli* based on the amino acid sequence as set forth in SEQ ID NO: 6 in the sequence listing, thereby synthesizing an artificial gene. The synthesized skp gene was amplified by PCR, using the primers (AAGGAGATATACATATGGATAAAATTGCCATTGTTAATAT (SEQ ID NO: 12), and TTGAGATCTGCCATATGTTATTTCACTTGTTTCAGAACG (SEQ ID NO: 13)), and the amplified gene was then cloned into MCS2 of the pETDue1 vector linearized with the restriction enzyme Ndel, using In-Fusion HD Cloning Kit, so as to obtain pETDuet_skp. Subsequently, the HER2-V2122 gene was incorporated into MCS1 of pETDuet_skp. Specifically, the artificially synthesized HER2-V2122 gene was amplified by PCR, using the primers (AGAAGGAGATATACCATGAAATATCTGCTGCCGAC (SEQ ID NO: 14), and CGCCGAGCTCGAATTTTAATGATGGTGATGATGATG (SEQ ID NO: 15)). Moreover, pETDuet_skp was linearized by PCR, using the primers (GGTATATCTCCTTCTTAAAGTTAAAC (SEQ ID NO: 16), and AATTCGAGCTCGGCGCGCCTGCAG (SEQ ID NO: 17)). The CEA-V2122 amplified by PCR and the linearized pETDuet_skp were subjected to cloning using In-Fusion HD Cloning Kit. The cloned vector was confirmed by sequencing, in terms of the gene sequence incorporated therein, and thereafter, it was referred to as pETDuet_HER2-V2122_skp.

For the expression of the protein, pETDuet_HER2-V2122_skp was transformed into BL21(DE3) (Nippon Gene Co., Ltd.), which was then pre-cultured in 2xYT medium (SIGMA-ADLRICH) at 37°C overnight. The medium used in the pre-culture was added to a new medium for 100-fold dilution, and culture was then carried out at 37°C, until OD (600 nm) became 0.5 to 2.0. Subsequently, IPTG was added to the culture to a final concentration of 0.5 mM, and the obtained mixture was then cultured at 37°C for 4 hours. Thereafter, a culture supernatant was recovered and was then preserved at 4°C.

The HER2-V2122 protein was roughly purified according to a batch method utilizing a 6xHis-Tag added to the C-terminus. Specifically, cOmplete His-Tag Purification Resin equilibrated with buffer A (50 mM Tris-HCl, 0.2 M NaCl, 1 mM EDTA, and 5 mM Imidazole; pH 8.0) was added to the culture supernatant preserved at 4°C. The obtained mixture was stirred for 2 hours to overnight at 4° C, so that the protein was allowed to bind to the resin. Subsequently, the resin was recovered into a column, and a 20 column volume of washing operation was performed with buffer A. Thereafter, a roughly purified product of HER2-V2122 was recovered by elution with buffer B (50 mM Tris-HCl, 0.2 M NaCl, 1 mM EDTA, and 400 mM Imidazole; pH 8.0).

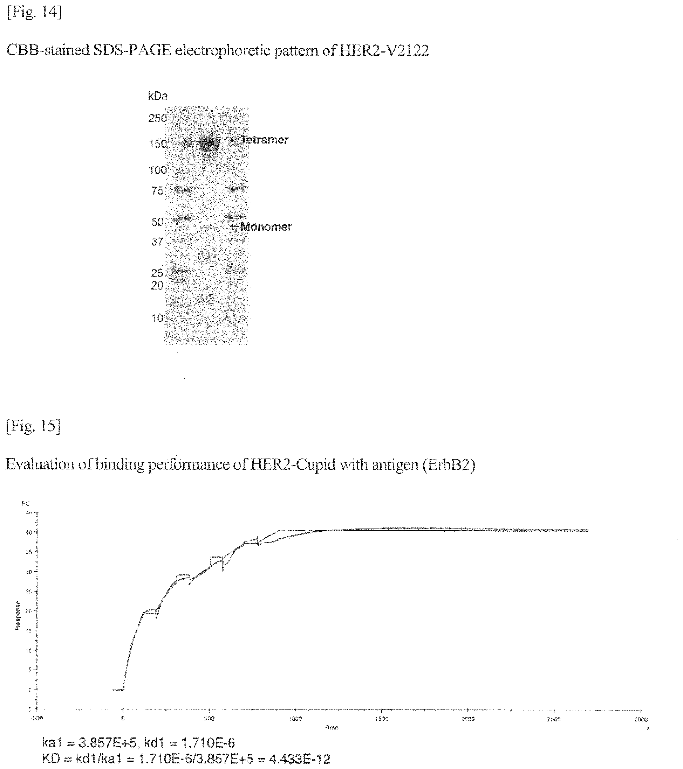

Subsequently, the roughly purified product was purified using a Protein L column. Specifically, 1 mL of Capto L (GE Healthcare) was filled into a PD-10 column and was then equilibrated with 10 column volume of PBS, and the aforementioned roughly purified product was then applied thereto. Thereafter, the resultant was washed with 10 column volume of PBS, was then eluted with 10 mM glycine hydrochloride (pH 2.0), and was then subjected to centrifugal concentration using Vivaspin Turbo 15 (MWCO 100,000). Moreover, using PD-10 (GE Healthcare), the buffer was replaced with PBS, and centrifugal concentration was further carried out using Vivaspin Turbo 4 (MWCO 100,000) to obtain a finally purified product. The purified product was subjected to CBB staining, and the purity of tetramer HER2-V2122 was assayed. The results are shown in Fig. 14. As an SDS-PAGE gel, Mini-PROTEAN TGX 4-15% (Bio-Rad) was used, and as a CBB staining solution, Bullet CBB Stain One (Ready To Use) (Nacalai Tesque, Inc.) was used.

From Fig. 14, it was confirmed that the purified HER2-V2122 comprises an approximately 150 kDa tetramer as a main component.

### Example 7: Evaluation of performance of HER2-V2122 by SPR

The affinity of HER2-V2122 for the antigen CEACAM5 was evaluated using a surface plasmon resonance (SPR) measuring device, Biacore T200 (GE Healthcare Life Sciences). Specifically, Recombinant Human ErbB2/Fc Chimera, Carrier Free (R & D SYSTEMS, 1129-ER-050) was immobilized on Sensor Chip CM5 (GE Healthcare Life Sciences) using an amine-coupling kit (GE Healthcare Life Sciences). The final amount of the ligand immobilized was 279 RU. Moreover, with regard to the purified HER2-V2122, two-fold serial dilutions from 1E-08 M to 6.25E-10 M were prepared as analytes. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.857E + 5, and kd1 = 1.710E - 6. Moreover, since evaluation can be carried out at K_{D} = kd1/ka1 in the bivalent analysis, the evaluation value K_{D} = kd1/ka1 = 1.710E - 6 / 3.857E + 5 = 4.433E - 12 was obtained. These results are shown in Fig. 15.

From the sensorgram and the calculated K_{D} value shown in Fig. 15, it was confirmed that HER-V2122 recognizes ErbB2 and strongly binds thereto.

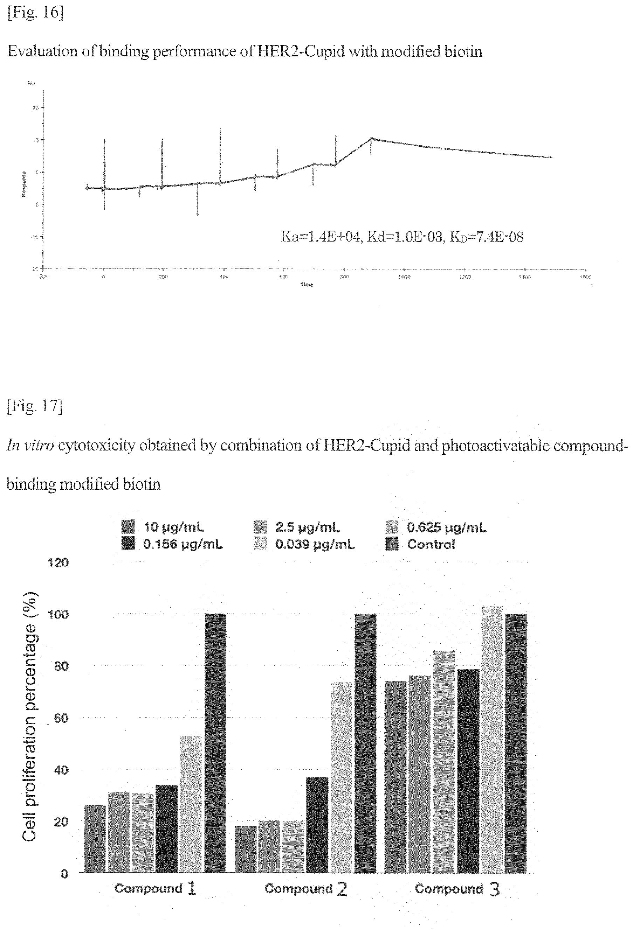

Furthermore, the interaction between HER2-V2122 and a modified biotin (Production Example 1, the compound represented by Formula 11) was also analyzed using Biacore T200. Specifically, an amine-coupling kit was used, a target value was set to be 5000 RU, and the purified CEA-V2122 was immobilized on Sensor Chip CM5. With regard to the concentrations of the analytes, 5 types of two-fold serial dilutions from 1E-08 M to 6.25E-10 M were used. Regarding interaction analysis, data were obtained by single-cycle kinetics analysis. Using Biacore T200 Evaluation Software, version 2.0, the obtained data were subjected to curve fitting in a bivalent analysis mode, and the evaluation value of affinity was obtained. These results are shown in Fig. 16.

### Example 8: In vitro cytotoxicity test using HER2-V2122 and photoactivatable compound-labeled modified biotins

A cytotoxicity test was carried out using the photoactivatable compound-labeled modified biotins, namely, Compound 1, Compound 2, and Compound 3. These compounds are described in Japanese Patent Application No. 2018-149295. Compound 1, Compound 2, and Compound 3 are shown in Fig. 7. Specifically, SK-BR-3 cells cultured in a McCoy's medium supplemented with 10% FBS were seeded on a 96-well plate for cell culture, so that the cell count became 5 x 10³ cells/well and the amount of the culture solution became 50 µL/well, and thereafter, the cells were cultured overnight. A solution containing a complex of CEA-V2122 and a photoactivatable compound-labeled modified biotin was prepared, so that the molar ratio of CEA-V2122 and each compound became 1 : 2, and the solution was then incubated at room temperature for 10 minutes. Thereafter, the concentration of the reaction solution was adjusted with a culture solution, so that the final concentration of HER2-V2122 became 10 µg/mL. Regarding serial dilutions, 20 µg/mL was set to be an initial concentration, from 4-fold serial dilutions (5.0 µg/mL, 1.25 µg/mL, 0.312 µg/mL, and 0.078 µg/mL), 5 complex serial dilution solutions were prepared. Besides, a medium alone that contained no complex was used as a zero control.

Such complex serial dilution solutions were each added in an amount of 50 µL/well to the cells cultured overnight, so that the final concentrations became 10 µg/mL, 2.5 µg/mL, 0.625 µg/mL, 0.156 µg/mL, and 0.039 µg/mL. One hour and two hours after addition of the complex, the cells were irradiated with a light, using LED emitting a light having a wavelength of 690 ± 10 nm, so that the irradiation energy became 100 J/cm². Thereafter, the cells were cultured for 48 hours, and thereafter, a comparison was made in terms of the number of surviving cells, using Cell Counting Kit-8 (DOJINDO LABORATORIES). Each condition was set to be n = 3. The dosage and administration were determined in accordance with the instruction manuals included with the kit, and addition of the reagent, the mixture was incubated for 1.5 hours at 37°C, in a CO₂ incubator. Thereafter, the absorbance at 450nm was measured, and the mean value was then calculated, followed by background collection. The control was set to be 100%, and the ratio of cell proliferation to the control under each condition was calculated. The results are shown in Fig. 17.

As shown in Fig. 17, it was confirmed that the complex of HER2-Cupid and a photoactivatable compound-labeled modified biotin exhibits cytotoxicity in a concentration-dependent manner.
SEQ ID NO: 1 
AEAGITGTWSDQLGDTFIVTAGADGALTGTYENAVGGAESRYVLTGRYDSAPATDGSGT

ALGWTVAWKNNSKNAHSATTWSGQYVGGADAKINTQWLLTSGTTNANAWKSTLVGH

DTFTKVKPSAASHHHHHH
SEQ ID NO: 2 (sm3E-scFv sequence) 
QVKLEQSGAEVVKPGASVKLSCKASGFNIKDSYMHWLRQGPGQRLEWIGWIDPENGDT

EYAPKFQGKATFTTDTSANTAYLGLSSLRPEDTAVYYCNEGTPTGPYYFDYWGQGTLVT
 
VSSGGGGSGGGGSGGGGSENVLTQSPSSMSVSVGDRVNIACSASSSVPYMHWLQQKPGK
SPKLLIYLTSNLASGVPSRFSGSGSGTDYSLTISSVQPEDAATYYCQQRSSYPLTFGGGTKL
EIK
SEQ ID NO: 3 
QVKLEQSGAEVVKPGASVKLSCKASGFNIKDSYMHWLRQGPGQRLEWIGWIDPENGDT
EYAPKFQGKATFTTDTSANTAYLGLSSLRPEDTAVYYCNEGTPTGPYYFDYWGQGTLVT
VSSGGGGSGGGGSGGGGSENVLTQSPSSMSVSVGDRVNIACSASSSVPYMHWLQQKPGK
SPKLLIYLTSNLASGVPSRFSGSGSGTDYSLTISSVQPEDAATYYCQQRSSYPLTFGGGTKL
EIKGGGGSGGGGAEAGITGTWSDQLGDTFIVTAGADGALTGTYENAVGGAESRYVLTGR
YDSAPATDGSGTALGWTVAWKNNSKNAHSATTWSGQYVGGADAKINTQWLLTSGTTN
ANAWKSTLVGHDTFTKVKPSAASHHHHHH
SEQ ID NO: 4 
MKYLLPTAAAGLLLLAAQPAMAQVKLEQSGAEVVKPGASVKLSCKASGFNIKDSYMH
WLRQGPGQRLEWIGWIDPENGDTEYAPKFQGKATFTTDTSANTAYLGLSSLRPEDTAVY
YCNEGTPTGPYYFDYWGQGTLVTVSSGGGGSGGGGSGGGGSENVLTQSPSSMSVSVGD
RVNIACSASSSVPYMHWLQQKPGKSPKLLIYLTSNLASGVPSRFSGSGSGTDYSLTISSVQ
PEDAATYYCQQRSSYPLTFGGGTKLEIKGGGGSGGGGAEAGITGTWSDQLGDTFIVTAG
ADGALTGTYENAVGGAESRYVLTGRYDSAPATDGSGTALGWTVAWKNNSKNAHSATT
WSGQYVGGADAKINTQWLLTSGTTNANAWKSTLVGHDTFTKVKPSAASHHHHHH
SEQ ID NO: 5 
ATGAAATATCTGCTGCCGACCGCAGCAGCGGGTCTGCTGCTGCTGGCAGCACAGCCT

GCAATGGCACAGGTTAAACTGGAACAGAGCGGTGCCGAAGTTGTTAAACCGGGTGC

AAGCGTTAAACTGAGCTGTAAAGCAAGCGGCTTTAACATCAAAGATAGCTATATGCA
 
TTGGCTGCGTCAGGGTCCGGGTCAGCGTCTGGAATGGATTGGTTGGATTGATCCGGA
AAATGGTGATACCGAATATGCACCGAAATTTCAGGGTAAAGCAACCTTTACCACCGA
TACCAGCGCAAATACCGCATATCTGGGTCTGAGCAGCCTGCGTCCGGAAGATACCGC
AGTGTATTATTGTAATGAAGGCACCCCGACCGGTCCGTATTATTTCGATTATTGGGGT
CAGGGCACCCTGGTTACCGTTAGCAGCGGTGGTGGTGGTAGTGGTGGCGGTGGTTCA
GGCGGTGGCGGTAGCGAAAATGTTCTGACCCAGAGCCCGAGCAGCATGAGCGTTAG
CGTTGGTGATCGTGTTAATATTGCATGTAGCGCAAGCAGCAGCGTTCCGTACATGCAC
TGGCTGCAGCAGAAACCGGGTAAAAGCCCGAAACTGCTGATTTATCTGACCAGCAAT
CTGGCAAGCGGTGTTCCGAGCCGTTTTAGCGGTAGCGGTAGTGGCACCGATTATAGC
CTGACCATTAGCAGCGTGCAGCCTGAAGATGCAGCAACCTATTATTGTCAGCAGCGT
AGCAGTTATCCGCTGACCTTTGGTGGTGGCACCAAACTGGAAATTAAAGGGGGTGGT
GGCTCAGGTGGCGGAGGTGCAGAAGCAGGTATTACCGGTACATGGTCAGATCAGCTG
GGTGATACCTTTATTGTTACCGCAGGCGCAGATGGTGCACTGACCGGCACCTATGAA
AATGCAGTTGGTGGTGCAGAAAGCCGTTATGTGCTGACCGGTCGTTATGATAGCGCA
CCGGCAACCGATGGTAGCGGCACCGCACTGGGTTGGACCGTTGCATGGAAAAATAAC
AGCAAAAATGCACATAGCGCAACCACCTGGTCAGGTCAGTATGTGGGTGGTGCCGAT
GCCAAAATTAACACCCAGTGGCTGCTGACCAGCGGTACAACCAATGCAAATGCCTGG
AAAAGTACCCTGGTTGGTCATGATACATTCACCAAAGTTAAACCGAGCGCAGCAAGC
CATCATCATCACCATCATTAA
SEQ ID NO: 6 
MDKIAIVNMGSLFQQVAQKTGVSNTLENEFKGRASELQRMETDLQAKMKKLQSMKAGS

DRTKLEKDVMAQRQTFAQKAQAFEQDRARRSNEERGKLVTRIQTAVKSVANSQDIDLV

VDANAVAYNSSDVKDITADVLKQVK
SEQ ID NO: 7 
EVQLVESGGGLVQPGGSLRLSCAASGFNIKDTYIHWVRQAPGKGLEWVARIYPTNGYTR
YADSVKGRFTISADTSKNTAYLQMNSLRAEDTAVYYCSRWGGDGFYAMDYWGQGTLV
TVSSGGGGSGGGGSGGGGSGGGGSDIQMTQSPSSLSASVGDRVTITCRASQDVNTAVAW
YQQKPGKAPKLLIYSASFLYSGVPSRFSGSRSGTDFTLTISSLQPEDFATYYCQQHYTTPPT
FGQGTKVEIK
SEQ ID NO: 8 
EVQLVESGGGLVQPGGSLRLSCAASGFNIKDTYIHWVRQAPGKGLEWVARIYPTNGYTR
YADSVKGRFTISADTSKNTAYLQMNSLRAEDTAVYYCSRWGGDGFYAMDYWGQGTLV
TVSSGGGGSGGGGSGGGGSGGGGSDIQMTQSPSSLSASVGDRVTITCRASQDVNTAVAW
YQQKPGKAPKLLIYSASFLYSGVPSRFSGSRSGTDFTLTISSLQPEDFATYYCQQHYTTPPT
FGQGTKVEIKGGGGGSGGGGGAEAGITGTWSDQLGDTFIVTAGADGALTGTYENAVGG
AESRYVLTGRYDSAPATDGSGTALGWTVAWKNNSKNAHSATTWSGQYVGGADAKINT
QWLLTSGTTNANAWKSTLVGHDTFTKVKPSAASHHHHHH
SEQ ID NO: 9 
MKYLLPTAAAGLLLLAAQPAMAEVQLVESGGGLVQPGGSLRLSCAASGFNIKDTYIHWV
RQAPGKGLEWVARIYPTNGYTRYADSVKGRFTISADTSKNTAYLQMNSLRAEDTAVYY
CSRWGGDGFYAMDYWGQGTLVTVSSGGGGSGGGGSGGGGSGGGGSDIQMTQSPSSLS
ASVGDRVTITCRASQDVNTAVAWYQQKPGKAPKLLIYSASFLYSGVPSRFSGSRSGTDFT
LTISSLQPEDFATYYCQQHYTTPPTFGQGTKVEIKGGGGSGGGGAEAGITGTWSDQLG
DTFIVTAGADGALTGTYENAVGGAESRYVLTGRYDSAPATDGSGTALGWTVAWKNNSK
NAHSATTWSGQYVGGADAKINTQWLLTSGTTNANAWKSTLVGHDTFTKVKPSAASHH
HHHH
SEQ ID NO: 10

ATGAAATATCTGCTGCCGACCGCAGCAGCGGGTCTGCTGCTGCTGGCAGCACAGCCT
GCAATGGCAGAAGTTCAGCTGGTTGAAAGCGGTGGTGGTCTGGTTCAGCCTGGTGGT
AGCCTGCGTCTGAGCTGTGCAGCAAGCGGTTTTAACATTAAAGATACCTATATTCATT
GGGTGCGTCAGGCACCTGGTAAAGGTCTGGAATGGGTTGCACGTATTTATCCGACCA
ATGGTTATACCCGTTATGCCGATAGCGTTAAAGGTCGTTTTACCATTAGCGCAGATAC
CAGCAAAAATACCGCATACCTGCAGATGAATAGTCTGCGTGCAGAGGATACCGCAGT
GTATTATTGTAGCCGTTGGGGTGGTGATGGTTTTTATGCAATGGATTATTGGGGTCAG
GGCACCCTGGTTACCGTTAGCTCAGGTGGAGGCGGTTCCGGTGGCGGAGGTTCCGGT
GGAGGTGGCTCCGGTGGCGGAGGTTCCGATATTCAGATGACCCAGAGTCCGAGCAGC
CTGAGCGCAAGCGTTGGTGATCGTGTGACCATTACCTGTCGTGCAAGCCAGGATGTT
AATACAGCAGTTGCATGGTATCAGCAGAAACCGGGTAAAGCACCGAAACTGCTGATT
TATAGCGCAAGCTTTCTGTATAGCGGTGTTCCGAGCCGTTTTAGCGGTAGCCGTAGCG
GCACCGATTTTACCCTGACCATTAGCAGCCTGCAGCCGGAAGATTTTGCAACCTATTA
TTGTCAGCAGCATTACACCACACCGCCTACCTTTGGCCAGGGCACCAAAGTTGAAAT
TAAAGGAGGTGGCGGTGGATCCGGCGGAGGTGGCGGAGCAGAAGCAGGTATTACCG
GTACATGGTCAGATCAGCTGGGTGATACCTTTATTGTTACCGCAGGCGCAGATGGTG
CACTGACCGGCACCTATGAAAATGCAGTTGGTGGTGCAGAAAGCCGTTATGTGCTGA
CCGGTCGTTATGATAGCGCACCGGCAACCGATGGTAGCGGCACCGCACTGGGTTGGA
CCGTTGCATGGAAAAATAACAGCAAAAATGCACATAGCGCAACCACCTGGTCAGGTC
AGTATGTGGGTGGTGCCGATGCCAAAATTAACACCCAGTGGCTGCTGACCAGCGGTA
CAACCAATGCAAATGCCTGGAAAAGTACCCTGGTTGGTCATGATACATTCACCAAAG
TTAAACCGAGCGCAGCAAGCCATCATCATCACCATCATTAA

## Claims

1. A fusion protein having the amino acid sequence as set forth in SEQ ID NO: 2 or SEQ ID NO: 7, a linker sequence, and the amino acid sequence as set forth in SEQ ID NO: 1 (provided that the amino acid sequence portion consisting of 6 histidine residues at the C-terminus thereof may be partially or entirely deleted), from the N-terminal side to the C-terminal side, in this order.

2. The fusion protein according to claim 1, wherein the number of amino acids of the linker sequence is 5 to 15.

3. The fusion protein according to claim 1 or 2, wherein the linker sequence consists of 4 to 14 glycine residues and 1 cysteine residue.

4. The fusion protein according to any one of claims 1 to 3, wherein the linker sequence is Gly-Gly-Gly-Gly-Ser-Gly-Gly-Gly-Gly or Gly-Gly-Gly-Gly-Gly-Ser-Gly-Gly-Gly-Gly-Gly.

5. A fusion protein having the amino acid sequence as set forth in SEQ ID NO: 3 or SEQ ID NO: 8 (provided that the amino acid sequence portion consisting of 6 histidine residues at the C-terminus thereof may be partially or entirely deleted).

6. The fusion protein according to any one of claims 1 to 5, further having a secretory signal sequence.

7. A fusion protein having the amino acid sequence as set forth in SEQ ID NO: 4 or SEQ ID NO: 9 (provided that the amino acid sequence portion consisting of 6 histidine residues at the C-terminus thereof may be partially or entirely deleted).

8. A nucleic acid encoding a fusion protein having the amino acid sequence as set forth in SEQ ID NO: 3 or SEQ ID NO: 8 (provided that the amino acid sequence portion consisting of 6 histidine residues at the C-terminus thereof may be partially or entirely deleted).

9. A nucleic acid encoding a fusion protein having the amino acid sequence as set forth in SEQ ID NO: 4 or SEQ ID NO: 9 (provided that the amino acid sequence portion consisting of 6 histidine residues at the C-terminus thereof may be partially or entirely deleted).

10. A cancer therapeutic agent or a cancer diagnostic agent, comprising the fusion protein according to any one of claims 1 to 7.

11. A kit for treating or diagnosing cancer, comprising (1) the fusion protein according to any one of claims 1 to 7, and (2) a conjugate of a compound represented by the following formula (1) or a salt thereof and a diagnostic substance or a therapeutic substance: 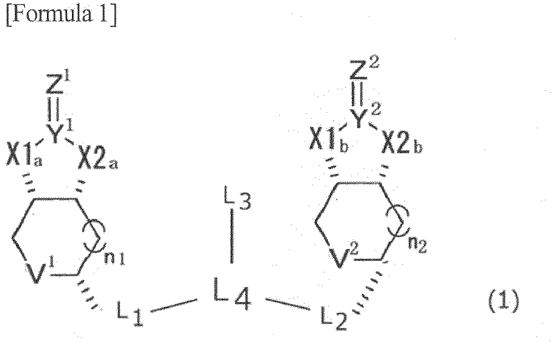 wherein
X1a, X1b, X2a and X2b each independently represent O or NH,
Y¹ and Y² each independently represent C or S,
Z¹ and Z² each independently represent O, S or NH,
V¹ and V² each independently represent S or S⁺-O⁻,
n1 and n2 each independently represent an integer of 0 or 1,
L₁ and L₂ each independently represent a divalent linking group,
L₃ represents a group comprising a functional group capable of binding to the diagnostic substance or the therapeutic substance at the terminus, and
L₄ represents a trivalent linking group.

12. The kit according to claim 11, wherein the diagnostic substance or the therapeutic substance is a phthalocyanine dye.
